# EUROPEAN PATENT SPECIFICATION

(11) **EP 2 760 458 B1**
(45) Date of publication and mention of the grant of the patent: **14.06.2017**
(21) Application number: 12770204.1
(22) Date of filing: 01.10.2012
(51) Int. Cl.: A61K 31/727, A61P 35/04

(54) **PREVENTION AND/OR TREATMENT OF CANCER AND/OR CANCER METASTASIS**
PRÄVENTION UND/ODER BEHANDLUNG VON KREBS UND/ODER KREBSMETASTASEN
PRÉVENTION ET/OU TRAITEMENT DU CANCER ET/OU D'UNE MÉTASTASE CANCÉREUSE

(30) Priority: 29.09.2011 GB 201116814; 07.02.2012 GB 201202081
(43) Date of publication of application: 06.08.2014
(73) Proprietor: The University of Liverpool, Liverpool, L69 7ZX (GB)
(72) Inventor: TURNBULL, Jeremy Ewan, Kidderminster Worcestershire, DY14 0NT (GB); YATES, Edwin Alexander, Southport PR8 4JS (GB); RHODES, Jonathan Michael, Malvern WR14 2JL (GB); YU, Lu-Gang, Liverpool L16 0JT (GB)
(74) Representative: HGF Limited
(86) International application number: PCT/GB2012/052428
(87) International publication number: WO 2013/045955

(56) References cited:
- WO-A1-2007/138263
- PATEY S J ET AL: "Engineered heparins: novel beta-secretase inhibitors as potential Alzheimer's disease therapeutics", NEURODEGENERATIVE DISEASES, KARGER AG, BASEL, CH, vol. 5, no. 3-4, 1 March 2008 (2008-03-01) , pages 197-199, XP008106568, ISSN: 1660-2854, DOI: 10.1159/000113701 [retrieved on 2008-03-06]
- GUIMOND S E ET AL: "Engineered bio-active polysaccharides from heparin", MACROMOLECULAR BIOSCIENCE, WILEY VCH VERLAG, WEINHEIM, DE, vol. 6, no. 8, 7 August 2006 (2006-08-07), pages 681-686, XP009164358, ISSN: 1616-5187 [retrieved on 2006-07-31]

## Description

### FIELD OF THE INVENTION

The present invention relates to the prevention and/or treatment of cancer and/or cancer metastasis. More specifically, the present invention relates to the prevention and/or treatment of cancer and/or cancer metastasis by inhibiting the activity of galectin-3.

### BACKGROUND OF THE INVENTION

Galectin-3 is a member of the galectin family of proteins. It is approximately 30 kDa in size and, like all galectins, contains a carbohydrate-recognition-binding domain (CRD) of about 130 amino acids that enables the specific binding of β-galactosides [Dumic et al., Biochim Biophys Acta 2006, 1760(4), 616-635; Liu et al., Biochim Biophys Acta 2002, 1572 (2-3), 263-273]. Galectin-3 is expressed in many types of human cells, in particular epithelial and immune cells, and it has been shown to be involved in a wide range of biological processes, including cell adhesion, cell activation and chemoattraction, cell growth and differentiation, cell cycle, and apoptosis [Dumic et al., Biochim Biophys Acta 2006, 1760(4), 616-635].

Galectin-3 is over-expressed and abnormally localized in many types of human cancers. As a consequence, it has attracted significant interest in cancer research over the past decades [Yu, World J Gastrointest Oncol 2010, 2(4), 177-180]. By way of example, galectin-3 has been associated with colorectal cancer [Watanabe et al., Oncology Reports 2011, 25, 1217-1226], thyroid cancer [Saussez et al., Thyroid 2008, 18(7), 705-712 and Isic et al., J Cancer Res Clin Oncol 2010, 136, 1805-1812], melanoma [Vereecken et al., Melanoma Research 2009, 19, 316-320; Vereecken et al., Clinical and Experimental Dermatology 2005, 31, 105-109], head and neck cancer [Saussez et al., Oral Oncology 2008, 44, 86-93], pancreatic cancer [Senapati et al., Clin Cancer Res 2011, 17, 267-274] lymphoma and myeloma [Hoyer et al., Am J Pathol 2004,164,893-902].

*In vitro* studies have also shown that accumulation of galectin-3 in the cytoplasm by stable galectin-3 gene transfection increases cancer cell invasion and promotes tumour angiogenesis [Califice et al., Oncogene 2004; 23, 7527-7536].

Accordingly, agents that inhibit the activity of galectin-3 are potentially useful for the targeted treatment of tumour angiogenesis, as well as cancers that are associated with galectin-3.

Metastasis is the primary cause of cancer-associated death. Adhesion of disseminating tumour cells to the blood vessel endothelium and homotypic aggregation of tumour cells to form tumour emboli in the circulation are crucial steps in the metastatic cascade (Pantel K et al., Nat Rev Cancer 2004, 4, 48-56; Miles et al., Clin Exp Metastasis 2008, 25, 305-24). Development of therapeutic agents that can specifically inhibit these metastatic steps could substantially enhance the survival of cancer patients.

Studies undertaken in the past few years have shown that the concentrations of circulating galectin-3 are markedly increased by up to 31-fold in the bloodstream of patients with breast, colorectal [Barrow et al, Clin Cancer Res 2011 Sep 20 (Epub ahead of print)], lung (Iurisci I et al., Clin Cancer Res 2000, 6, 1389-1393), bladder (Sakaki et al., J Med Invest. 2008, 55, 127-132), pharyngeal cancers (Saussez et al., Oral Onco 2008, 44, 86-93) and melanoma (Vereecken et al., Clin Exp Dermatol 2006, 31, 105-109). Patients with metastatic disease are also found to have higher concentrations of circulating galectin-3 than those with localized tumours (Yu, World J Gastrointest Oncol 2010, 2, 177-180). Until very recently, the functional implication, if any, of the increased circulation of galectin-3 in cancer patients remained unknown.

However, it is now known that free circulating galectin-3 plays an important role in promoting tumour cell metastatic spread by enhancing tumour cell heterotypic adhesion to vascular endothelium (Zhao et al., Cancer Res 2009, 69, 6799-6806) and by enhancing the homotypic aggregation of cancer cells to form microemboli in the circulation (Zhao et al., Mol Cancer 2010, 9, 154). These effects are mediated in part by the interaction of galectin-3 with the oncofetal Galβ1,3GalNAc- (TF) antigen on the cancer-associated transmembrane mucin protein MUC1 (Yu et al., J Biol Chem 2007, 282, 773-781). It has been shown that this effect of galectin-3 results from the cell surface polarization of the large transmembrane glycoprotein MUC1 and the consequent exposure of underlying cell surface adhesion molecules including E-Cadherin, CD44 and unidentified ligands to endothelial-associated E-selectin.

Earlier investigations have also shown that cancer cell-surface-associated galectin-3 may also play an important role in cancer metastasis by acting directly as an adhesion molecule (Takenaka et al., Glycoconj J. 2004, 19, 543-549). It increases cancer cell invasion at primary tumour sites by interaction with the basement matrix proteins and enhances cancer cell invasion by interaction with galactoside-terminated glycans on the surface of vascular endothelium (see review by Liu and Rabinovich, Nat Rev Cancer 2005, 5, 29-41). Furthermore, suppression of galectin-3 expression in human colon cancer cells using anti-sense technology before inoculation of the tumour cells into experimental mice is associated with reduced tumour cell liver colonization and metastasis (Bresalier et al., Gastroenterology 1998, 115, 287-296).

Thus, targeting galectin-3 activity in the circulation may hold significant promise for future development of novel therapeutic agents to prevent metastasis and reduce cancer-associated fatality [Yu, World J Gastrointest Oncol 2010, 2(4), 177-180].

It is an object of the present invention to provide inhibitors of galactin-3 activity that are potentially useful agents for the treatment of cancer and/or cancer metastasis.

### SUMMARY OF THE INVENTION

The present invention resides in the recognition that certain heparin derivatives (as defined hereinafter) act as inhibitors of galectin-3 activity. Accordingly, the heparin derivatives of the present invention are potentially useful agents for the prevention and/or treatment of cancer and/or cancer metastasis. Such cancers could include cancers of solid organs such as, but not exclusively, lung, breast, colon, rectum, pancreas, liver and ovary but could also include cancers of blood, bone marrow or lymphoid tissue including, but not exclusively lymphoma and multiple myeloma.

Thus, in a first aspect, the present invention provides a heparin derivative as defined herein, or a pharmaceutically acceptable salt thereof, for use in the prevention and/or treatment of cancer and/or cancer metastasis.

In a further aspect, the present invention provides a method of preventing and/or treating cancer and/or cancer metastasis, the method comprising administering to a subject in need of such treatment a therapeutically effective amount of a heparin derivative as defined herein, or a pharmaceutically acceptable salt thereof.

In a further aspect, the present invention provides the use of a heparin derivative as defined herein, or a pharmaceutically acceptable salt thereof, in the manufacture of a medicament for use in the prevention and/or treatment of cancer and/or cancer metastasis.

In another aspect, the present invention provides a pharmaceutical composition comprising a heparin derivative as defined herein, or a pharmaceutically acceptable salt thereof, in admixture with a pharmaceutically acceptable diluent or carrier. Suitably, the pharmaceutical composition is for use in the treatment of cancer and/or cancer metastasis.

Without wishing to be bound by any particular theory, it is believed that the heparin derivatives of the present invention exert their therapeutic effects, at least in part, by inhibiting the activity of galectin-3. In particular, the heparin derivatives of the present invention have been found to inhibit the interaction between galectin-3 and MUC1 transmembrane proteins and inhibit galectin-3-induced cancer cell adhesion to the endothelial cells. It is for this reason that these derivatives have been identified as potentially useful agents for the treatment of cancer metastasis.

In a further aspect, the present invention provides a heparin derivative as defined herein, or a pharmaceutically acceptable salt thereof, for use in the inhibition of galectin-3 activity.

In a further aspect, the present invention provides the use of a heparin derivative as defined herein, or a salt thereof, as an inhibitor of galectin-3 activity *(in vitro* or *in vivo*).

In a further aspect, the present invention provides a method of inhibiting the activity of galectin-3 (*in vitro* or *in vivo*), the method comprising administering an effective amount of a heparin derivative as defined herein, or a pharmaceutically acceptable salt thereof.

In yet another aspect, the present invention provides a heparin derivative which is obtainable by / obtained by / directly obtained by any one of the methods defined herein.

Further aspects of the invention are outlined in the following detailed description of the invention.

### BRIEF DESCRIPTION OF THE FIGURES

Figure 1 shows the effects of the heparin derivatives described in Example 1 herein on galectin-3 binding to TF-expressing asialo bovine mucin. The extent of galectin-3 binding was quantified by ELISA.
Figure 2 shows the effects of the heparin derivatives described in Example 1 herein on the inhibition of galectin-3-mediated adhesion of human colon cancer HT29-5F7 cells to monolayer of human micro-vascular lung endothelial cells (HMVEC-Ls) in culture.
Figure 3 shows the anticoagulant activity (anti-Xa activity) of the heparin derivatives described in Example 1 herein.
Figure 4 shows the binding of galectin-3 to BSA, anti-freeze glycoprotein, asialofeutin, and asialo bovine mucin. The extent of galectin-3 binding was quantified by ELISA.
Figure 5 is a Table showing the disaccharide composition of the heparin derivatives prepared in Example 1. In Figure 5, P1=A, standard heparin; P2=B, *N*-acetylated; P3=C, 2-de-*O*-sulfated; P4=E: 2-de-*O*-sulfated and *N*-acetylated; P5=D, 6-de-*O*-sulfated; P6=F, 6-de-O-sulfated and *N*-acetylated; P7=G, 2-,6-de-*O*-sulfated and *N*-sulfated; P8=H, 2-,6-de-*O*-sulfated and *N*-acetylated.
Figure 6 shows an elution profile for the digested heparin derivative E (compound E) prepared following the free-radical digestion (as described in Example 5). The separation was performed using a GE Healthcare XK26/100 Sephadex G100 column (bed volume 450 ml) running isocratically with 500 mM ammonium bicarbonate. The Y-axis shows the UV absorbance at 232nM.
Figure 7 shows the inhibition of galectin-3 (1µg/ml) binding to TF-expressing glycan asialo-fetuin (when assessed by ELISA) for the heparin derivatives/compounds C, C1, C2, C3, D, D1, D2, D3, E, E3, F, F1, F2, F3, G, H, H1, H2 and H3 at 40 µg/ml.
Figures 8A-D show the dose-dependent inhibition of galectin-3 (1µg/ml) binding to asialo-fetuin by heparin derivatives/compounds E, E3, F and F3 at 2 -500µg/ml.
Figures 9 and 10 show the dose-dependent inhibition of galectin-3 (1µg/ml)-induced adhesion of human melanoma ACA19+ cells to a HUVEC (Human Umbilical Vein Endothelial Cell) monolayer by heparin derivatives E and E3 (Figure 9) and F and F3 (Figure 10) at 1 - 100µg/ml.
Figure 11 shows the inhibition of galectin-3-mediated trans-endothelial invasion of human melanoma ACA19+ cells through HUVEC monolayer for heparin derivatives/compounds C, D, E, F and G at 100µg/ml.
Figures 12 and 13 show the inhibition of galectin-3 (1µg/ml)-mediated trans-endothelial invasion of human melanoma ACA19+ cells through a HUVEC (Human Umbilical Vein Endothelial Cell) monolayer for heparin derivatives E and E3 (Figure 12) and F3 (Figure 13) at 10-100µg/ml.
Figures 14A-C show effects of the heparin derivatives/compounds C, C1, C2, C3, D, D1, D2, D3, E1, E2, E3, F, F1, F2, F3, G, G1, G2, G3, H, H1, H2, and H3 (at 30µg/ml) on P- (Figure 14A), L- (Figure 14B) and E- (Figure 14C) selectin binding to SLeX.
Figure 15 shows the anti-factor IIa activity of the heparin derivatives/compounds C, C1, C2, C3, D, D1, D2, D3, E1, E2, E3, F, F1, F2, F3, G, G1, G2, G3, H, H1, H2, and H3 at a concentration of 100µg/ml.
Figures 16A and 16B show the cytotoxicity of the heparin derivatives/compounds C, C1, C2, C3, D, D1, D2, D3, E1, E2, E3, F, F1, F2, F3, G, G1, G2, G3, H, H1, H2, and H3 to cancer cells (SW620 cells; Figure 16A) and endothelial cells (HUVEC cells; Figure 16B) following the experimental procedures outlined in Example 11.

### DETAILED DESCRIPTION OF THE INVENTION

### Definitions

Unless otherwise stated, the following terms used in the specification and claims have the following meanings set out below.

It is to be appreciated that references to "preventing" or "prevention" relate to prophylactic treatment and includes preventing or delaying the appearance of clinical symptoms of the state, disorder or condition developing in a human that may be afflicted with or predisposed to the state, disorder or condition but does not yet experience or display clinical or subclinical symptoms of the state, disorder or condition.

It will be appreciated that references to "treatment" or "treating" of a state, disorder or condition includes: (1) inhibiting the state, disorder or condition, *i.e.,* arresting, reducing or delaying the development of the disease or a relapse thereof (in case of maintenance treatment) or at least one clinical or subclinical symptom thereof, or (2) relieving or attenuating the disease, *i.e.* causing regression of the state, disorder or condition or at least one of its clinical or subclinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

The term "alkyl" includes both straight and branched chain alkyl groups as well as cycloalkyl groups. References to individual alkyl groups such as "propyl" are specific for the straight chain version only and references to individual branched chain alkyl groups such as "isopropyl" are specific for the branched chain version only. For example, "(1-8C)alkyl" includes (1-4C)alkyl, (1-3C)alkyl, propyl, isopropyl, *t*-butyl, cyclohexyl, cyclopentyl, cyclobutyl and cyclopropyl. In a particular embodiment, an "alkyl" group is a (1-8C) alkyl group. In another embodiment, an "alkyl" group is a (1-6C)alkyl group.

A "substituted alkyl" group is an alkyl group bearing one or more substituent groups. In an embodiment, the substituent groups are selected from halo, amino, hydroxy, nitro, cyano, (1-6C)alkoxy, aryl, (2-6C)acyl, amido or phosphate groups.

The term "aryl" means a cyclic or polycyclic aromatic ring having from 6 to 12 carbon atoms. Examples of aryl groups include, but are not limited to, phenyl, biphenyl, naphthyl and the like. In particular embodiment, an aryl group is phenyl.

The term "substituted aryl" refers to aryl groups bearing one or more substituent groups. In an embodiment, the substituent groups are selected from halo, amino, hydroxy, nitro, cyano, (1-6C)alkyl, (1-6C)alkoxy, (2-6C)acyl, or amido groups.

The term "acyl" is used herein to refer to groups of the formula R^{a}-CO-, wherein R^{a} is selected from (1-8C)alkyl or aryl. In an embodiment, an acyl group is (2-6C)acyl group, such as acetyl, pentanoyl or pivaloyl. In another embodiment, the acyl group is an aryl-acyl group, such as a benzoyl or phthaloyl group. In a preferred embodiment, the acyl group is acetyl.

The term "substituted acyl" is used herein to refer to an acyl group substituted with one or more suitable substituent groups. In an embodiment, an acyl group is substituted with one or more halo atoms, such a fluorine, chlorine or bromine. Particular examples of substituted acyl groups include mono-, di- or tri-fluoroacetyl groups.

The term "halo" refers to fluoro, chloro, bromo and iodo.

The term "amido" is used herein to refer to a group of the formula -CONH₂.

The term "substituted amido" is used herein to refer groups of the formula -CONR^{b}R^{c} or -NR^{b}CO-R^{c}, wherein R^{b} is hydrogen or (1-6C)alkyl and R^{c} is selected from substituted or unsubstiuted (1-6C)alkyl or substituted or unsubstiuted aryl. Examples of substituted amido groups include methylamido, ethylamido or phthalamido groups.

Where substituents are chosen from "one or more" groups it is to be understood that this definition includes all substituents being chosen from one of the specified groups or the substituents being chosen from two or more of the specified groups.

### Heparin derivatives

Heparin is widely used clinically as an anticoagulant. The anticoagulant activity of heparin arises from its ability to increase the rate of formation of irreversible complexes between antithrombin III and the serine protease clotting factors Xₐ and IIa. However, attenuation of the anticoagulant activity of heparin is vital if its derivatives are to be developed for use as pharmaceuticals for different therapeutic applications.

Unfractionated and low molecular weight heparins are highly sulphated glycosaminoglycan having a molecular weight typically ranging from 3kDa to 30kDa. It consists of 1,4 linked disaccharide repeat units of α-L-iduronic or β-D-glucuronic acid linked to either *N-*acetyl or *N*-sulpho-α-D-glucosamine. The principal positions of *O*-sulphation are C-2 of the uronate moieties and C-6 of the glucosamine moieties as well as, more rarely, C-3 of the glucosamine moiety. The most common disaccharide unit present in heparin is composed of a 2-O-sulphated iduronic acid and a 6-O-sulphated, N-sulphated glucosamine. However, variable substitution during biosynthesis does result in considerable sequence diversity.

The present invention relates to the use of particular heparin derivatives.

More specifically, the heparin derivatives of the present invention comprise uronate moieties that are substantially 2-O desulphated and/or glucosamine moieties that are substantially 6-O desulphated. The heparin derivatives of the present invention advantageously possess little or no anticoagulant activity.

Therefore, the heparin derivatives of the present invention are comprised of one or more disaccharide units, each disaccharide unit comprising a uronate moiety linked to a glucosamine moiety, wherein the 2-*O* atom of the uronate moiety and/or the 6-*O* atom of the glucosamine moiety are substantially desulphated.

By "substantially 2-O and/or 6-O desulphated", we mean that 10 to 100% of the 2-O atoms on the uronate moieties and/or the 6-O atoms of the glucosamine moieties of the native heparin molecule are desulphated, i.e. the sulphate groups are replaced with hydrogen atoms.

In an embodiment, 30 to 100% of the 2-O atoms on the uronate moieties and/or the 6-O atoms of the glucosamine moieties of the heparin molecule are desulphated, i.e. the sulphate groups are replaced with hydrogen atoms.

In a further embodiment, 50 to 100% of the 2-O atoms on the uronate moieties and/or the 6-O atoms of the glucosamine moieties of the heparin molecule are desulphated, i.e. the sulphate groups are replaced with hydrogen atoms.

In a further embodiment, 75 to 100% of the 2-O atoms on the uronate moieties and/or the 6-O atoms of the glucosamine moieties of the heparin molecule are desulphated, i.e. the sulphate groups are replaced with hydrogen atoms.

In a further embodiment, 90 to 100% of the 2-O atoms on the uronate moieties and/or the 6-O atoms of the glucosamine moieties of the heparin molecule are desulphated, i.e. the sulphate groups are replaced with hydrogen atoms.

In a further embodiment, 90 to 95% of the 2-O atoms on the uronate moieties and/or the 6-O atoms of the glucosamine moieties of the heparin molecule are desulphated, i.e. the sulphate groups are replaced with hydrogen atoms.

In a further embodiment, 95 to 100% of the 2-O atoms on the uronate moieties and/or the 6-O atoms of the glucosamine moieties of the heparin molecule are desulphated, i.e. the sulphate groups are replaced with hydrogen atoms.

In one embodiment, the heparin derivatives of the present invention may be substantially 2-N-sulphated on the glucosamine moiety (e.g. greater than 70% or 75% sulphated).

In an alternative embodiment, the heparin derivatives of the invention are substantially 2-N desulphated on the glucosamine. Thus, the heparin derivatives of the present invention may comprise glucosamine residues that are substantially 2-N desulphated on the glucosamine in addition to having uronate moieties that are substantially 2-O desulphated and/or glucosamine moieties that are substantially 6-O desulphated.

By "substantially 2-N desulphated", we mean that 10 to 100% of the 2-N atoms of the glucosamine moieties of the native heparin molecule are desulphated, i.e. the sulphate groups present on the 2-N atoms are replaced with hydrogen atoms or a substituent group as defined herein. In an embodiment, 30 to 100% of the 2-N atoms of the glucosamine moieties of the native heparin molecule are desulphated. In a further embodiment, 50 to 100% of the 2-N atoms of the glucosamine moieties of the native heparin molecule are desulphated. In yet another embodiment, 70 to 100%, 75 to 100%, 90 to 100%, 90 to 95% or 95 to 100% of the 2-N atoms of the glucosamine moieties of the native heparin molecule are desulphated.

Suitable substituents for the 2-N atom of the glucosamine moiety are any organic or inorganic chemical group other than sulphate (SO₃). In an embodiment, the 2-N atom of the glucosamine moieties are substituted with a substituent selected from the group consisting of hydrogen, substituted or unsubstituted alkyl, substituted or unsubstituted aryl, substituted or unsubstituted acyl, substituted or unsubstituted amido and phosphate.

The 2-N alkyl group may be linear, branched or cyclic and is preferably a (1-6C) alkyl group, optionally substituted with one or more atoms or groups, such as halogen atoms (e.g. fluorine, chlorine or bromine) or aryl, acyl, amido or phosphate groups.
The amido group bonded directly to the 2-N glucosamine atom and/or the amido group bonded to the alkyl group bonded to the 2-N glucosamine atom may take any convenient form, such as a methylamido, ethylamido or phthalamido group.
The substituted or unsubstituted acyl group bonded directly to the 2-N glucosamine atom and/or the acyl group bonded to the alkyl group bonded to the 2-N glucosamine atom may be linear (e.g. pentanoyl) or branched (e.g. pivaloyl) and is preferably a
1-6C substituted or unsubstituted acyl group. The acyl group may be an arylacyl group, such as a benzoyl group. The acyl group may be substituted with one or more halogen atoms, particularly fluorine, chlorine or bromine atoms. Preferred N-acyl groups are mono-, di- and tri-fluoroacetyl group. A further preferred N-acyl group is
a phthaloyl group. Preferably the 2-N glucosamine atom is substituted with an acyl group selected from the group consisting of substituted or unsubstituted acetyl, substituted or unsubstituted proprionyl and substituted or unsubstituted butanoyl.
Most preferably the glucosamine 2-N atom is substituted with an unsubstituted acetyl group.

In a particular embodiment of the invention, the heparin derivatives are:
(i) substantially 2-O desulphated and substantially 2-N desulphated (e.g. 2-N substituted) as defined herein;
(ii) substantially 6-O desulphated and substantially 2-N desulphated (e.g. 2-N substituted) as defined herein;
(iii) substantially 2-O desulphated and substantially 6-O desulphated as defined herein; or
(iv) substantially 2-O desulphated, substantially 6-O desulphated, and substantially 2-N desulphated (e.g. 2-N substituted) as defined herein.

In a further embodiment of the invention, the heparin derivatives are substantially 2-O desulphated and substantially 2-N desulphated (e.g. 2-N substituted) as defined herein. In such an embodiment, the level of 2-O and 2-N desulphation may be any of the levels defined previously herein. In a specific embodiment, the level of 2-O desulphation is 85 to 100% and the level of 2-N desulphation is 70 to 100%. In another embodiment, the level of 2-O desulphation is 90 to 100%; the level of 2-N desulphation is 75 to 100%. The level of 6-O sulphation in such embodiments may be, for example, between 70 to 100% or between 75 to 100%.

In a further embodiment of the invention, the heparin derivatives are substantially 6-O desulphated and substantially 2-N desulphated (e.g. 2-N substituted as defined hereinbefore). In such an embodiment, the level of 6-O and 2-N desulphation may be any of the levels defined previously herein. In a specific embodiment, the level of 6-O desulphation is 85 to 100% and the level of 2-N desulphation is 70 to 100%. In another embodiment, the level of 6-O desulphation is 90 to 100% and the level of 2-N desulphation is 90 to 100% or 95 to 100%. The level of 2-O sulphation in such embodiments may be, for example, between 70 to 100% or between 75 to 100%.

In a particular embodiment of the invention, the heparin derivatives are substantially 2-O desulphated and substantially 6-O desulphated as defined herein. In such an embodiment, the level of 2-O and 6-O desulphation may be any of the levels defined previously herein. In a specific embodiment, the level of 2-O and 6-O desulphation is 85 to 100%. In another embodiment, the level of 2-O and 6-O desulphation is 90 to 100%. The level of 2-N sulphation in such embodiments may be, for example, between 70 to 100% or between 75 to 100%.

In a further embodiment of the invention, the heparin derivatives are substantially 2-O, 6-O and 2-N desulphated (e.g. 2-N substituted) as defined herein. In such an embodiment, the level of 2-O, 6-O and 2-N desulphation may be independently any of the levels defined previously herein. In a specific embodiment, the level of 2-O and 6-O desulphation is 70 to 100% and the level 2-N desulphation is 90 to 100% or 95 to 100%. In another embodiment, the level of 2-O desulphation is 70 to 100%, the level of 6-O desulphation is 85 to 100% and the level of 2-N desulphation is 90 to 100% or 95 to 100%.

The average molecular weight of the heparin derivatives of the invention will range from 500 Da to 20 kDa. Suitably, the average molecular weight of the heparin derivatives of the invention will range from 500 Da to 15 kDa. In an embodiment, the average molecular weight of the heparin derivatives is from 2 kDa to 15 kDa. In a further embodiment, the average molecular weight of the heparin derivatives is from 10 kDa to 15 kDa.

Suitably, the average molecular weight of the heparin derivatives of the invention will range from about 500 Da to about 3.5 kDa, and more suitably from about 500 Da to about 3 kDa.

The degree of polymerisation of the heparin derivatives of the present will range from 2 monomer units (i.e. a disaccharide) up to 60 monomer units.

In an embodiment, the degree of polymerisation of the heparin derivatives of the present will range from 4 monomer units up to 60 monomer units.

In a further embodiment, the degree of polymerisation of the heparin derivatives of the present will range from 10 monomer units up to 60 monomer units.

In a further embodiment, the degree of polymerisation of the heparin derivatives of the present will range from 2 monomer units up to 7 monomer units, or from 3 monomer units up to 6 monomer units.

In an embodiment, the heparin derivatives of the invention will comprise a mixture of different size heparin derivatives. In such cases, the average degree of polymerisation of the heparin derivatives will range from 2 monomer units (i.e. a disaccharide) up to 60 monomer units, and more suitably 4-60, 2 to 10, 2 to 7 or 3 to 6 monomer units.

In an embodiment, the heparin derivatives of the present invention may be represented by the general structural formula I shown below: 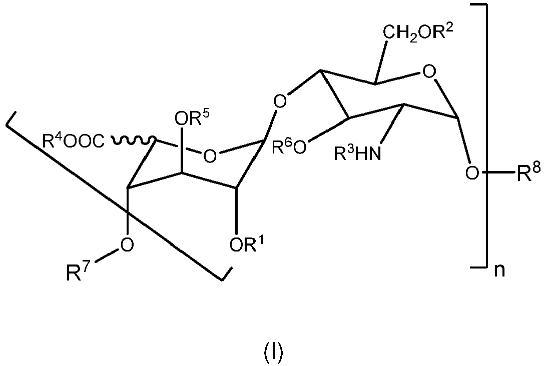 wherein:
R¹ and R² are selected from hydrogen or sulphate, with the proviso that either:
   (i) substantially all of the R¹ groups present in the molecule are hydrogen when substantially all (e.g. >70%) of the R² groups present are sulphate;
   (ii) substantially all of the R² groups present in the molecule are hydrogen when substantially all (e.g. >70%) of the R¹ groups present are sulphate, or
   (iii) substantially all of the R¹ and R² groups present in the molecule are hydrogen;
n is 1 to 30;
R³ is selected from sulphate, hydrogen, substituted or unsubstituted alkyl, substituted or unsubstituted aryl, substituted or unsubstituted acyl, substituted or unsubstituted amido and phosphate;
R⁴, R⁵ and R⁶ are each separately selected from the group consisting of hydrogen, sulphate, phosphate, substituted or unsubstituted (1-6C)alkyl, substituted or unsubstituted (1-6C)alkoxy, substituted or unsubstituted aryl, substituted or unsubstituted aryloxy, substituted or unsubstituted acyl, and substituted or unsubstituted amido; and R⁷ and R⁸ are each separately selected from the group consisting of hydrogen, substituted or unsubstituted alkyl, substituted or unsubstituted aryl, substituted or unsubstituted acyl, a terminal monosaccharide group, a terminal disaccharide group and/or fragments or derivatives thereof;
or a pharmaceutically acceptable salt thereof.

The bond shown as 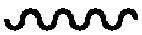 in formula I above is intended to show that the group -COOR⁴ may be above (as in (β-D)glucuronate moiety) or below (as in (α-L)iduronate moiety) the plane of the ring. It will be appreciated that the uronate moiety may also be a (α-L)galacturonate (although this is not shown).

In an embodiment, substantially all R¹ groups present are hydrogen and substantially all (e.g. >70%) R² groups present are sulphate.

In an alternative embodiment, substantially all R¹ groups present are sulphate and substantially all (e.g. >70%) R² groups present are hydrogen.

In a further alternative embodiment, substantially all R¹ and R² groups present are hydrogen.

Particular heparin derivatives of this embodiment of the invention include, for example, compounds of Formula I, or pharmaceutically acceptable salts thereof, wherein, unless otherwise stated, each of R¹, R², R³, R⁴, R⁵, R⁶, R⁷, and R⁸ has any of the meanings defined hereinbefore or in any of paragraphs (1) to (55) hereinafter:-
(1) n is 1;
(2) n is 2 to 30;
(3) n is 2 to 20;
(4) n is 2 to 10;
(5) n is 2 to 7;
(6) n is 3 to 6;
(7) between 10-100% of all R¹ groups present are hydrogen when substantially all (e.g >70%) of the R² groups present are sulphate;
(8) between 30-100% of all R¹ groups present are hydrogen when substantially all (e.g >70%) of the R² groups present are sulphate;
(9) between 50-100% of all R¹ groups present are hydrogen when substantially all (e.g >70%) of the R² groups present are sulphate;
(10) between 75-100% of all R¹ groups present are hydrogen when substantially all (e.g >70%) of the R² groups present are sulphate;
(11) between 90-100% of all R¹ groups present are hydrogen when substantially all (e.g >70%) of the R² groups present are sulphate;
(12) between 90-95% of all R¹ groups present are hydrogen when substantially all (e.g >70%) of the R² groups present are sulphate;
(13) between 10-100% of all R² groups present are hydrogen when substantially all (e.g >70%) of the R¹ groups present are sulphate;
(14) between 30-100% of all R² groups present are hydrogen when substantially all (e.g >70%) of the R¹ groups present are sulphate;
(15) between 50-100% of all R² groups present are hydrogen when substantially all (e.g >70%) of the R¹ groups present are sulphate;
(16) between 75-100% of all R² groups present are hydrogen when substantially all (e.g >70%) of the R¹ groups present are sulphate;
(17) between 90-100% of all R² groups present are hydrogen when substantially all (e.g >70%) of the R¹ groups present are sulphate;
(18) between 90-95% of all R² groups present are hydrogen when substantially all (e.g >70%)of the R¹ groups present are sulphate;
(19) between 10-100% of all R¹ and R² groups present are hydrogen;
(20) between 30-100% of all R¹ and R² groups present are hydrogen;
(21) between 50-100% of all R¹ and R² groups present are hydrogen;
(22) between 75-100% of all R¹ and R² groups present are hydrogen;
(23) between 90-100% of all R¹ and R² groups present are hydrogen;
(24) between 90-95% of all R¹ and R² groups present are hydrogen;
(25) R³ is sulphate;
(26) R³ is hydrogen, substituted or unsubstituted (1-8C)alkyl, substituted or unsubstituted aryl, substituted or unsubstituted (2-8C)acyl, substituted or unsubstituted amido or phosphate;
(27) R³ is hydrogen, unsubstituted (1-6C)alkyl, unsubstituted aryl, or unsubstituted (2-6C)acyl;
(28) R³ is hydrogen, (1-4C)alkyl, phenyl, or acetyl;
(29) R³ is hydrogen;
(30) R³ is acetyl;
(31) between 10-100% of all R³ groups present are hydrogen or a substituent group other than sulphate;
(32) between 30-100% of all R³ groups present are hydrogen or a substituent group other than sulphate;
(33) between 50-100% of all R³ groups present are hydrogen or a substituent group other than sulphate;
(34) between 70-100% of all R³ groups present are hydrogen or a substituent group other than sulphate;
(35) between 75-100% of all R³ groups present are hydrogen or a substituent group other than sulphate;
(36) between 90-100% of all R³ groups present are hydrogen or a substituent group other than sulphate;
(37) between 95-100% of all R³ groups present are hydrogen or a substituent group other than sulphate;
(38) R⁴ is selected from the group consisting of hydrogen, substituted or unsubstituted (1-6C)alkyl, or substituted or unsubstituted aryl;
(39) R⁴ is selected from the group consisting of hydrogen, unsubstituted (1-6C)alkyl, or unsubstituted aryl;
(40) R⁴ is hydrogen or sulphate;
(41) R⁴ is hydrogen;
(42) R⁵ is selected from the group consisting of hydrogen, sulphate, phosphate, substituted or unsubstituted (1-6C)alkyl, substituted or unsubstituted (1-6C)alkoxy, substituted or unsubstituted aryl, substituted or unsubstituted aryloxy, substituted or unsubstituted acyl, and substituted or unsubstituted amido;
(43) R⁵ is selected from the group consisting of hydrogen, sulphate, phosphate, unsubstituted (1-6C)alkyl, unsubstituted (1-6C)alkoxy, unsubstituted aryl, sunsubstituted aryloxy, unsubstituted (2-6C)acyl, and substituted or unsubstituted amido;
(44) R⁵ is hydrogen or sulphate;
(45) R⁵ is hydrogen;
(46) R⁶ is selected from the group consisting of hydrogen, sulphate, phosphate, substituted or unsubstituted (1-6C)alkyl, substituted or unsubstituted (1-6C)alkoxy, substituted or unsubstituted aryl, substituted or unsubstituted aryloxy, substituted or unsubstituted acyl, and substituted or unsubstituted amido;
(47) R⁶ is selected from the group consisting of hydrogen, sulphate, phosphate, unsubstituted (1-6C)alkyl, unsubstituted (1-6C)alkoxy, unsubstituted aryl, sunsubstituted aryloxy, unsubstituted (2-6C)acyl, and substituted or unsubstituted amido;
(48) R⁶ is hydrogen or sulphate;
(49) R⁶ is hydrogen;
(50) R⁶ is sulphate;
(51) R⁷ and R⁸ are each separately selected from the group consisting of hydrogen, substituted or unsubstituted (1-6C)alkyl, substituted or unsubstituted aryl, substituted or unsubstituted (2-6C)acyl, a terminal monosaccharide group, or a terminal disaccharide group;
(52) R⁷ and R⁸ are each separately selected from the group consisting of hydrogen, unsubstituted (1-6C)alkyl, unsubstituted aryl, unsubstituted (2-6C)acyl, a terminal monosaccharide group, or a terminal disaccharide group;
(53) R⁷ and R⁸ are both hydrogen;
(54) one of R⁷ and R⁸ is a mono- or di-saccharide moiety and the other is hydrogen;
(55) both of R⁷ and R⁸ are mono- or di-saccharide moieties.

In a particular embodiment, R⁴ and R⁵ are both hydrogen.

In a further embodiment, R⁴, R⁵ and R⁶ are all hydrogen.

In yet another embodiment, R¹ and R² are selected from hydrogen or sulphate, with the proviso that either: (i) substantially all of R¹; (ii) substantially all of R², or (iii) substantially all of R¹ and R² are hydrogen; R³ is acyl (e.g. acetyl) or sulphate; R⁴, R⁵ and R⁶ are all hydrogen; and R⁷ and R⁸ have any one of the definitions set out herein.

In yet another embodiment, R¹ and R² are selected from hydrogen or sulphate, with the proviso that substantially all of R¹, or substantially all of R², or substantially all of R¹ and R² are hydrogen; R³ is acyl (e.g. acetyl) or sulphate; R⁴, R⁵, R⁶, R⁷ and R⁸ are all hydrogen.

In an embodiment, substantially all (e.g. >70%) of the R³ groups in the molecule are sulphate.

In an alternative embodiment, substantially all (e.g. >70%) of the R³ groups present are hydrogen or a substituent as defined above other than sulphate.

In a group of heparin derivatives:
(i) substantially all of the R¹ groups are hydrogen (i.e. the molecule is 2-O desulphated as defined hereinbefore) and substantially all of the R³ groups present are hydrogen or a substituent other than sulphate as defined above (i.e. the molecule is 2-N desulphated (e.g. 2-N substituted) as defined hereinbefore);
(ii) substantially all of the R² groups are hydrogen (i.e. the molecule is 6-O desulphated as defined hereinbefore) and substantially all of the R³ groups present are hydrogen or a substituent other than sulphate as defined above (i.e. the molecule is 2-N desulphated (e.g. 2-N substituted) as defined hereinbefore);
(iii) substantially all of the R¹ and R² groups are hydrogen (i.e. the molecule is substantially 2-O and 6-O desulphated as defined herein); or
(iv) substantially all of the R¹ and R² groups are hydrogen (i.e. the molecule is substantially 2-O and 6-O desulphated as defined herein) and substantially all of the R³ groups present are hydrogen or a substituent other than sulphate as defined above (i.e. the molecule is 2-N desulphated (e.g. 2-N substituted) as defined hereinbefore).

In a particular embodiment, substantially all of the R¹ groups are hydrogen (i.e. the molecule is 2-O desulphated as defined hereinbefore) and substantially all of the R³ groups present are hydrogen or a substituent other than sulphate as defined above (i.e. the molecule is 2-N desulphated (e.g. 2-N substituted) as defined hereinbefore). In such an embodiment, the level of 2-O and 2-N desulphation may be any of the levels defined previously herein. In a specific embodiment, the level of 2-O desulphation is 85 to 100% (i.e. 85 to 100% of the R¹ groups are hydrogen) and the level of 2-N desulphation is 70 to 100% (i.e. 70 to 100% of the R³ groups are hydrogen or a substituent group other than sulphate as defined herein). In another embodiment, the level of 2-O desulphation is 90 to 100% (i.e. 90 to 100% of the R¹ groups are hydrogen); the level of 2-N desulphation is 75 to 100% (i.e. 75 to 100% of the R³ groups are hydrogen or a substituent group other than sulphate as defined herein). In such embodiments, the level of 6-O sulphation may be, for example, between 70 to 100% or between 75 to 100% (i.e. 70 to 100% or 75 to 100% of the R² groups are sulphate and the remainder may be hydrogen).

In a further embodiment of the invention, substantially all of the R² groups are hydrogen (i.e. the molecule is 6-O desulphated as defined hereinbefore) and substantially all of the R³ groups present are hydrogen or a substituent other than sulphate as defined above (i.e. the molecule is 2-N desulphated (e.g. 2-N substituted) as defined hereinbefore). In such an embodiment, the level of 6-O and 2-N desulphation may be any of the levels defined previously herein. In a specific embodiment, the level of 6-O desulphation is 85 to 100% (i.e. 85 to 100% of the R² groups are hydrogen) and the level of 2-N desulphation is 70 to 100% (i.e. 70 to 100% of the R³ groups are hydrogen or a substituent group other than sulphate as defined herein). In another embodiment, the level of 6-O desulphation is 90 to 100% (i.e. 90 to 100% of the R² groups are hydrogen) and the level of 2-N desulphation is 90 to 100 or 95 to 100% (i.e. 90 to 100% or 95 to 100% of the R³ groups are hydrogen or a substituent group other than sulphate as defined herein). The level of 2-O sulphation in such embodiments may be, for example, between 70 to 100% or between 75 to 100% (i.e. 70 to 100% or 75 to 100% of the R¹ groups are sulphate and the remainder may be hydrogen).

In a particular embodiment of the invention, substantially all of the R¹ and R² groups are hydrogen (i.e. the molecule is substantially 2-O and 6-O desulphated as defined herein). In such an embodiment, the level of 2-O and 6-O desulphation may be any of the levels defined previously herein. In a specific embodiment, the level of 2-O and 6-O desulphation is 85 to 100% (i.e. 85 to 100% of the R¹ and R² groups are hydrogen). In another embodiment, the level of 2-O and 6-O desulphation is 90 to 100% (i.e. 90 to 100% of the R¹ and R² groups are hydrogen). The level of 2-N sulphation in such embodiments may be, for example, between 70 to 100% or between 75 to 100% (i.e. 70 to 100% or 75 to 100% of the R³ groups are sulphate).

In a further embodiment of the invention, substantially all of the R¹ and R² groups are hydrogen (i.e. the molecule is substantially 2-O and 6-O desulphated as defined herein) and substantially all of the R³ groups present are hydrogen or a substituent other than sulphate as defined above (i.e. the molecule is 2-N desulphated (e.g. 2-N substituted) as defined hereinbefore).

In such an embodiment, the level of 2-O, 6-O and 2-N desulphation may be independently any of the levels defined previously herein. In a specific embodiment, the level of 2-O and 6-O desulphation is 70 to 100% (i.e. 70 to 100% of the R¹ and R² groups are hydrogen) and the level 2-N desulphation is 90 to 100% or 95 to 100% (i.e. 90 to 100% or 95 to 100% of the R³ groups are hydrogen or a substituent group other than sulphate as defined herein). In another embodiment, the level of 2-O desulphation is 70 to 100% (i.e. 70 to 100% of the R¹ groups are hydrogen), the level of 6-O desulphation is 85 to 100% (i.e. 85 to 100% of the R² groups are hydrogen) and the level of 2-N desulphation is 90 to 100% or 95 to 100% (i.e. 90 to 100% or 95 to 100% of the R³ groups are hydrogen or a substituent group other than sulphate as defined herein).

It will also be appreciated that where the compound of the present invention consists solely of a saccharide unit of Formula (I) in which one of R⁷ and R⁸ is hydrogen and the other one of R⁷ and R⁸ is a terminal monosaccharide, the compound as a whole will consist of an odd number of monosaccharide units, whereas, if R⁷ and R⁸ are the same (i.e. R⁷ and R⁸ are both hydrogen, monosaccharides or disaccharides) then the compound will consist of an even number of monosaccharide units. Moreover, if one of R⁷ and R⁸ is a monosaccharide and the other of R⁷ and R⁸ is a disaccharide then the compound will consist of an odd number of monosaccharides. Thus, Formula (I) above, and the other structural formulae presented herein, are all intended to encompass compounds containing both odd and even numbers of monosaccharide units.

Where R⁷ is a terminal monosaccharide group it is preferred that R⁷ is a glucosamine moiety or derivative or fragment thereof. R⁷ may take the same structure as the glucosamine moiety in Formula (I) in which R², R³ and R⁴ are as defined above.

Where R⁷ is a terminal disaccharide group, R⁷ suitably has the structure of the bracketed disaccharide repeating unit such that the non-reducing terminal monosaccharide has the same general structure as the uronate moiety in Formula (I), i.e. an (α-L)iduronate, (β-D)glucuronate or (α-L)galacturonate moiety in which R¹, R⁴ and R⁵ have any one of the definitions set out herein. The disaccharide unit R⁷ may include derivatives of one or both of the monosaccharides forming part of the bracketed disaccharide repeating unit. The non-reducing terminal monosaccharide may be an (α-L)iduronate, (β-D)glucuronate or (α-L)galacturonate moiety incorporating a Δ4-5 unsaturated ring (i.e. a C-to-C double bond between carbons 4 and 5 in the ring). Such unsaturation arises, for example, when polysaccharide fragments forming the compound are made by digestion with a bacterial lyase enzyme or a chemical beta-elimination process (commonly used to fragment heparin).

Where R⁸ is a terminal monosaccharide group, R⁸ is preferably a uronate moiety or derivative or fragment thereof. R⁸ preferably has the same general structure as the uronate moiety in Formula (I), i.e. an (α-L)iduronate, (β-D)glucuronate or (α-L)galacturonate moiety in which R¹, R⁴ and R⁵ are as defined above.

Where R⁸ is a terminal disaccharide group, R⁸ preferably has the same general structure to the bracketed disaccharide repeat of Formula (I) such that the reducing terminal monosaccharide, may have the same structure as the glucosamine moiety in Formula (I) in which R², R³ and R⁴ are as defined above. Disaccharide unit R⁸ may include derivatives of one or both of the monosaccharides forming part of the bracketed disaccharide repeating unit. The reducing terminal monosaccharide may be 2,5-anhydro-mannitol, a 2,5-anhydromannose residue, a 1,6 anhydro (bicyclic) ring structure, or a mannosamine residue. Production of the compound may involve nitrous acid digestion, in which case the reducing terminal monosaccharide R7is likely to be 2,5-anhydro-mannitol, which is normally chemically reduced to a 2,5-anhydromannose residue. Production of the compound using a chemical beta elimination process, in which case some reducing terminal residues can also be found as a 1,6 anhydro (bicyclic) structure, generally derived from 6-O-sulphated glucosamine residues. In addition, the chemical beta-elimination process can also cause epimerisation of glucosamine residues to form mannosamine residues.

Thus, in an embodiment, and in reference to Formula (I) above, the compound may be represented by one of the following three preferred structures (Formula (II), (III) and (IV)) in which all R groups and n are as defined above in relation to Formula (I), and the uronate moiety is represented generally as an (α-L)iduronate or (β-D)glucuronate moiety for convenience, but could be a (α-L)galacturonate moiety instead.

In Formula (II), R⁷ is a terminal glucosamine moiety of the same general structure as that included in the bracketed disaccharide repeating unit.

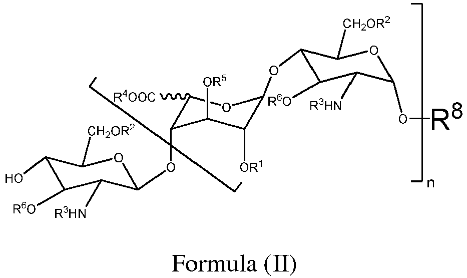

In Formula (III), R⁸ is a terminal uronate moiety of the same general structure as that included in the bracketed disaccharide repeating unit.

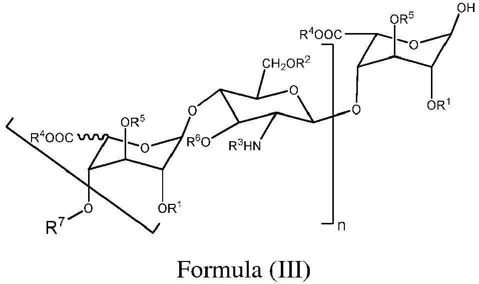

In Formula (IV), R⁷ is a terminal glucosamine moiety of the same general structure as that included in the bracketed disaccharide repeating unit and R⁸ is a terminal uronate moiety of the same general structure as that included in the bracketed disaccharide repeating unit.

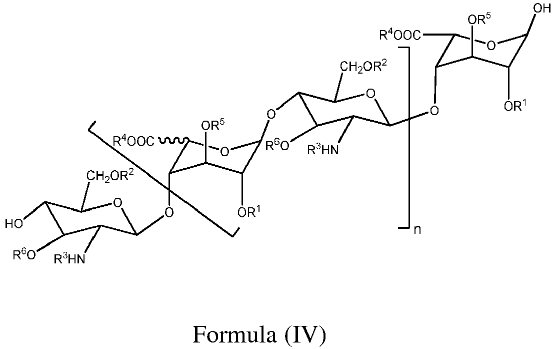

Particular heparin derivatives/compounds of the invention include the derivatives/compounds C, C1, C2, C3, D, D1, D2, D3, E1, E2, E3, F, F1, F2, F3, G, G1, G2, G3, H, H1, H2, and H3 defined herein, or a pharmaceutically acceptable salt or solvate thereof.

### Anticoagulant activity

With regard to anti-coagulant activity, it is preferred that the heparin derivatives of the present invention exhibits less than around 20 % of the Anti-Factor Xa activity of unmodified porcine intestinal mucosal heparin (PIMH). Preferably the heparin derivatives exhibits less than around 5 %, more preferably less than around 1 % of the Anti-Factor Xa activity of unmodified PIMH. It is particularly preferred that the heparin derivatives of the invention exhibits less than about 0.5 %, still more preferably less than about 0.2 % of the Anti-Factor Xa activity of unmodified PIMH.

Anti Factor Xa activity can be measured against a porcine mucosal heparin (PIMH) standard of known activity (Sigma, UK) using a diagnostic grade Coatest Heparin test kit (Chromogenix, MA), adapted to a 96-well plate format, reading A405 (Polarstar plate reader (BMG LabTechnologies, U.K.)) [Patey et al., J Med Chem. 2006, 49, 6129-6132].

Suitably, the heparin derivatives of the present invention show little or no Factor IIa activity, or little or no activity in Activated Partial Thromboplastin Time (APTT) and PT assays.

In reference to Example 10 herein, the exemplified heparin derivatives of the present invention show no detectable activity against Factor Xa and Factor IIa at concentrations that are 100 fold higher than the IC₅₀ of heparin. In addition, with the exception of the exemplified heparin derivatives D and D1, the exemplified heparin derivatives of the present invention also show no detectable inhibition at concentrations 100 fold higher than the IC₅₀ of heparin in the APTT assay described in Example 10. Furthermore, in the PT assay, the heparin derivatives of the present invention show no activity at 100 µg/ml.

### Cancer metastasis treatment

For the treatment of cancer metastasis, the heparin derivative is a 2-O and/or 6-O desulphated heparin derivative as defined hereinbefore, i.e. it may have any one of the definitions set out above.

### Cancer treatment

For the treatment of cancer, the heparin derivatives suitably have less than around 1 % of the Anti-Factor Xa activity of unmodified PIMH. More suitably, the heparin derivatives possess less than or equal to 0.5 % of the Anti-Factor Xa activity of unmodified PIMH.

In a further embodiment, the heparin derivatives of the present invention for use in the treatment of cancer comprise glucosamine moieties that are substantially 2-N desulphated as defined hereinbefore. In a particular embodiment, the 2-N atom of the glucosamine moiety is substituted with hydrogen, substituted or unsubstituted alkyl, substituted or unsubstituted aryl, substituted or unsubstituted acyl, substituted or unsubstituted amido or phosphate in place of sulphate.

In particular embodiment, the heparin derivatives for the treatment of cancer are of formula I as defined herein, wherein R³ is hydrogen, substituted or unsubstituted alkyl, substituted or unsubstituted aryl, substituted or unsubstituted acyl, substituted or unsubstituted amido or phosphate and all of the other substituent groups have any one of the definitions set out herein.

For the treatment of cancer/tumours (rather than cancer metastasis), the heparin derivatives of the present invention are either: (i) substantially 2-O desulphated; or (ii) substantially 2-O desulphated and 6-O desulphated heparin derivatives as defined herein. In other words, the heparin derivatives in this embodiment are preferably not just O-6 desulphated.

In one embodiment, the heparin derivatives for the treatment of cancer are substantially 2-O desulphated and 6-O sulphated. By "6-O sulphated", we mean that greater than 70% of the 6-O atoms of the glucosamine moieties of the heparin molecule are sulphated. The level of 2-O desulphation on the uronate moieties may be 30 to 100%, 50 to 100%, 75 to 100%, 90 to 100% or 90 to 95% as defined hereinbefore.

In a further embodiment, the heparin derivative for the treatment of cancer is substantially 2-O desulphated and 6-O desulphated. The level of 2-O and 6-O desulphation may be 30 to 100%, 50 to 100%, 75 to 100%, 90 to 100% or 90 to 95% as defined hereinbefore.

In a further embodiment, the heparin derivatives for use in the treatment of cancer are of formula I above, wherein R¹ and R² are selected from hydrogen or sulphate, with the proviso that either (i) substantially all of the R¹ groups present are hydrogen (i.e. are desulphated) when substantially all of the R² groups (>70%) are sulphate, or (ii) substantially all of the R¹ and R² groups present are hydrogen (i.e. are desulphated).

In a further embodiment, the heparin derivatives of the present invention for use in the treatment of cancer comprise uronate moieties that are substantially 2-O desulphated and/or glucosamine moieties that are substantially 6-O desulphated and 2-N desulphated as defined hereinbefore.

A suitable pharmaceutically acceptable salt of a heparin derivative of the invention is, for example, an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

It is also to be understood that the heparin derivatives of the invention may exist in solvated as well as unsolvated forms such as, for example, hydrated forms. It is to be understood that the invention encompasses all such solvated forms that possess the desired anti-cancer and/or anti-metastatic activity.

The *in vivo* effects of a heparin derivative of the invention may be exerted in part by one or more metabolites that are formed within the human or animal body after administration.

### Preparation of heparin derivatives

The heparin derivatives of the present invention can be prepared from native heparin using techniques well known in the art.

The key chemical modifications required in order to produce the heparin derivatives of the present invention from native heparin include: the fragmentation of heparin molecules to produce derivatives of varying size fractions; chemical modification to remove sulphate groups at the 2-O position in the uronate moieties and/or the 6-O position of the glucosamine moieties; and chemical modification to provide N-sulphated or N-acylated glucosamine moieties.

Particular examples of techniques that may be utilised to form the heparin derivatives of the present invention from native heparin include:
(i) selective removal of 2-O sulphate from the uronate moieties;
(ii) selective removal of 6-O sulphate from the glucosamine moieties;
(iii) complete removal of O- and N-sulphates;
(iv) selective removal of N-sulphates;
(v) addition of N-sulphates (re-N-sulphation); and
(vi) acylation of N groups (or re-N-acylation).

All of the above reactions can be carried out by techniques known in the art. Suitable examples of such techniques are described further in, for example, WO 2007/138263 and Patey et al., J. Med. Chem. 2006, 49, 6129-6132, the entire contents of which are incorporated herein by reference.

By way of example, reaction (i) above, i.e. the selective removal of 2-O sulphate groups from the uronate moieties, can be carried out by the techniques described in Jaseja et al. [Can. J. Chem. 1989, 67, 1449-1456]. Reaction (ii) above, i.e. the selective removal of 6-O sulphate from the glucosamine moieties, can be carried out by the techniques described in Inoue et al. [Anal. Biochem. 1975, 65, 164-174]. Reaction (iii) above, i.e. the complete removal of O- and N-sulphates, can be carried out by the techniques described in Inoue et al. [Anal. Biochem. 1975, 65, 164-174]. Reaction (iv) above, i.e. the selective removal of N-sulphates, can be carried out by employing solvolytic de-sulphation under kinetic control, as described by Inoue et al. [Carbohydr. Res. 1976, 46, 87-95]. Reaction (v) above, i.e. the addition of N-sulphates (re-N-sulphation), can be carried out by the use of trimethylamine-sulfur trioxide using the methodology described in Lloyd et al. [Biochem. Pharmacol. 1971, 20, 637-648]. Lastly, reaction (vi) above, i.e. acylation of N groups (or re-N-acylation), can be achieved using the techniques described in Yates et al. [Carbohydr. Res. 1996, 294, 15-27].

An example of a suitable native heparin source to use as a starting material is porcine mucosal heparin (PIMH).

It will also be appreciated that the heparin derivatives described herein can also be produced by other methods. Synthetic chemistry approaches have been described for production of such derivatives, such as the heparin pentasaccharide Arixtra [US Patent 4,818,816; Angew. Chem. Int. Ed. 2004, 43, 3118] and also other heparin and heparan sulfate structures [J. Am. Chem. Soc. 2009, 128, 2766-2767; J Am Chem Soc. 2006, 131, 17394-405; Chemistry. 2010, 26, 8365-75; Cole CL et al. PloS One 2010, 53-7; Nature Chemistry 2011, 3, 557-563]. Enzymatic and chemoenzymatic approaches have also been described using purified or recombinant enzymes involved in the natural biosynthesis of heparin/heparan sulfate to modify oligosaccharide or polysaccharide substrates produced from bacterial fermentation or synthetic chemistry [J. Biol. Chem. 2005, 280, 42817; Chem. Biol. 2007, 14, 986; J. Am. Chem. Soc. 2008, 130, 12998**;** J. Med. Chem. 2005, 48, 349; J. Biol. Chem. 2010, 285, 34240; Nat. Biotechnol. 2003, 21, 1343].

In the preparation of the heparin derivatives of the invention, all proposed reaction conditions, including choice of solvent, reaction atmosphere, reaction temperature, duration of the experiment and workup procedures etc., can be selected by a person skilled in the art.

It is understood by one skilled in the art that the functionality present on various portions of the molecule must be compatible with the reagents and reaction conditions utilised.

### Pharmaceutical compositions

A further aspect of the present invention provides a pharmaceutical composition for use in the prevention and/or treatment of a cancer and/or cancer metastasis comprising a heparin derivative as defined hereinbefore, or pharmaceutically acceptable salt thereof, in combination with a pharmaceutically acceptable carrier, diluent or excipient.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular, intraperitoneal or intramuscular dosing) or as a suppository for rectal dosing.

Suitably, the compositions of the present invention will be suitable for parenteral delivery.

The compositions of the invention may be obtained by conventional procedures using conventional pharmaceutical excipients, well known in the art. Thus, compositions intended for parenteral use may contain, for example, a suitable vehicle and, optionally, one or more preservative agents.

It will be appreciated that a suitable vehicle used for the heparin derivatives of the invention should be one which is well tolerated by the subject to whom it is given and enables delivery of the heparin derivative to the desired site of action.

In one embodiment, the amount of the heparin derivative of the invention in a pharmaceutical composition of the invention is an amount from about 0.01 mg to about 800 mg. In another embodiment, the amount of the heparin derivative is an amount from about 0.01 mg to about 500 mg. In another embodiment, the amount of the heparin derivative is an amount from about 0.01 mg to about 250 mg. In another embodiment, the amount of the heparin derivative is an amount from about 0.1 mg to about 60 mg. In another embodiment, the amount of the heparin derivative is an amount from about 1 mg to about 20 mg.

### Routes of administration and dosage

The heparin derivatives of the invention may be administered in a number of ways depending on the desired site of action.

Suitably, the heparin derivative will be administered by injection. Injections may be, for example, intravenous, intra-arterial, intradermal, subcutaneous, intraperitoneal, intracerebral, intracerebroventricular or intrathecal. Such injections may be continuous over a period of time (infusion) or bolus injections.

The heparin derivatives may also be administered by inhalation (e.g. intranasally), orally, transdermally or by the rectal or vaginal routes in certain circumstances.

The heparin derivatives of the invention may also be incorporated into a slow or delayed release formulation or device. Such formulations or devices may, for example, be inserted on or under the skin and the heparin derivative may be released over weeks or even months.

It will be appreciated that the amount of the heparin derivative of the invention required is determined by biological activity and bioavailability which in turn depends on the mode of administration, the physicochemical properties of the derivative employed and whether the derivative is being used as a monotherapy or in a combined therapy. The frequency of administration will also be influenced by the above mentioned factors and particularly the half-life of the derivative within the subject being treated.

Optimal dosages of the heparin derivative to be administered may be determined by those skilled in the art, and will vary with the particular derivative being used, the strength of the preparation, the mode of administration, and the advancement of the disease condition. Additional factors depending on the particular subject being treated will result in a need to adjust dosages, including subject age, weight, gender, diet, and time of administration.

Known procedures, such as those conventionally employed by the pharmaceutical industry (e.g. *in vivo* experimentation, clinical trials, etc.), may be used to establish specific formulations of the heparin derivatives as well as precise dosage regimens.

Generally, a daily dose of between 0.01 µg/kg of body weight and 1.0 g/kg of body weight of the heparin derivative may be used for the treatment of cancer and/or cancer metastasis, depending upon which specific derivative is used. More preferably, the daily dose is between 0.01 mg/kg of body weight and 100 mg/kg of body weight.

Daily doses may be given as a single administration (e.g. as a single daily injection or infusion). Alternatively, the compound used may require administration two or more times during a day.

### Therapeutic uses and applications

### Mechanism of Action

As previously stated, and without wishing to be bound by any particular theory, it is believed that the heparin derivatives of the present invention exert their therapeutic effects, at least in part, by inhibiting the activity of galectin-3.

Thus, in one aspect, the present invention provides a heparin derivative as defined herein, or a pharmaceutically acceptable salt thereof, for use in the inhibition of galectin-3 activity.

In a further aspect, the present invention provides the use of a heparin derivative as defined herein, or a salt thereof, as an inhibitor of galectin-3 activity (*in vitro*).

In a further aspect, the present invention provides a method of inhibiting the activity of galectin-3 (*in vitro* or *in vivo*), the method comprising administering an effective amount of a heparin derivative as defined herein, or a pharmaceutically acceptable salt thereof.

In particular, the heparin derivatives of the present invention have been found to inhibit the interaction between galectin-3 and MUC1 transmembrane proteins and also to inhibit galectin-3-induced cancer cell adhesion to the endothelial cells. It is for this reason that these derivatives have been identified as potentially useful agents for the treatment of cancer metastasis.

Thus, the present invention also provides:
- A heparin derivative as defined herein, or a pharmaceutically acceptable salt thereof, for use in the inhibition of galectin-3 mediated binding of cancer cells to endothelial cells.
- The use of a heparin derivative as defined herein, or a salt thereof, as an inhibitor of galectin-3 mediated binding of cancer cells to endothelial cells.
- A method of inhibiting the galectin-3 mediated binding of cancer cells to endothelial cells (*in vitro* or *in vivo*) by administering a heparin derivative as defined herein, or a pharmaceutically acceptable salt thereof.
- A heparin derivative as defined herein, or a pharmaceutically acceptable salt thereof, for use in the inhibition of the interaction between galectin-3 and MUC1 transmembrane proteins.
- The use of a heparin derivative as defined herein, or a salt thereof, as an inhibitor of the interaction between galectin-3 and MUC1 transmembrane proteins *(in vitro*).
- A method of inhibiting the activity of galectin-3 binding to MUC1 transmembrane proteins *(in vitro* or *in vivo*), the method comprising administering an effective amount of a heparin derivative as defined herein, or a pharmaceutically acceptable salt thereof.
- A heparin derivative as defined herein, or a pharmaceutically acceptable salt thereof, for use in the inhibition of galectin-3-mediated cancer metastasis.
- The use of a heparin derivative as defined herein, or a salt thereof, as an inhibitor of galectin-3 activity *(in vitro*).
- A method of inhibiting the activity of galectin-3 (*in vitro* or *in vivo*), the method comprising administering an effective amount of a heparin derivative as defined herein, or a pharmaceutically acceptable salt thereof.
- A heparin derivative as defined herein, or a pharmaceutically acceptable salt thereof, for use in the inhibition of galectin-3 mediated binding of cancer cells to endothelial cells.
- The use of a heparin derivative as defined herein, or a salt thereof, as an inhibitor of galectin-3 mediated binding of cancer cells to endothelial cells.
- A method of inhibiting the galectin-3 mediated binding of cancer cells to endothelial cells (*in vitro* or *in vivo*) by administering a heparin derivative as defined herein, or a pharmaceutically acceptable salt thereof.
- A heparin derivative as defined herein, or a pharmaceutically acceptable salt thereof, for use in the inhibition of the interaction between galectin-3 and MUC1 transmembrane proteins.
- A heparin derivative as defined herein, or a salt thereof, as an inhibitor of the interaction between galectin-3 and MUC1 transmembrane proteins *(in vitro*).
- A method of inhibiting the activity of galectin-3 binding to MUC1 transmembrane proteins (*in vitro* or *in vivo*), the method comprising administering an effective amount of a heparin derivative as defined herein, or a pharmaceutically acceptable salt thereof.

The heparin derivatives may also exert their therapeutic effects by one more alternative mechanisms of action (that do not involve the inhibition of galectin-3 and/or the interaction between galectin-3 and MUC1).

Thus, in another aspect, the invention also encompasses a heparin derivative as defined herein, or a salt thereof, for use as an inhibitor of the galectin-3-mediated, MUC1-independent, cancer adhesion and metastasis.

### Treatment of cancer

The galectin-3 inhibitory activity demonstrated by the heparin derivatives of the present invention render them suitable for the prevention and/or treatment of cancer.

Thus, the present invention also provides:
- A heparin derivative as defined herein, or a pharmaceutically acceptable salt thereof, for use in the prevention and/or treatment of cancer.
- A method of preventing and/or treating cancer, the method comprising administering to a subject in need of such treatment a therapeutically effective amount of a heparin derivative as defined herein, or a pharmaceutically acceptable salt thereof.
- The use of a heparin derivative as defined herein, or a pharmaceutically acceptable salt thereof, in the manufacture of a medicament for use in the prevention and/or treatment of cancer.

Cancers suitable for treatment with the heparin derivatives of the present invention include:
- colorectal cancer [Watanabe et al., Oncology Reports, 2011, 25, 1217-1226; Barrow et al, Clin Cancer Res, 2011 Sep 20 (electronic publication)];
- head and neck cancer [Saussez et al., Oral Oncology, 2008, 44, 86-93];
- pancreatic cancer [Senapati et al., Clin Cancer Res, 2011, 17, 267-274];
- breast cancer [Iurisci et al, Clin Cancer Res, 2000, 6,1389-93];
- lung cancer [Iurisci et al, Clin Cancer Res, 2000, 6,1389-93];
- melanoma [Vereecken et al., Melanoma Research, 2009, 19, 316-320; Vereecken et al., Clinical and Experimental Dermatology, 2005, 31, 105-109];
- thyroid cancer [Saussez et al., Thyroid, 2008, 18(7), 705-712 and Isic et al., J Cancer Res Clin Oncol, 2010, 136, 1805-1812].
- Bladder cancer [Sakaki et al, J Med Invest, 2008, 55, 127-132]

Galectin-3 activity has also been associated with tumour angiogenesis [Califice *et al., Oncogene* 2004; 23, 7527-7536]. Accordingly, the heparin derivatives of the present invention are also potentially useful agents for the prevention and/or treatment of tumour angiogenesis.

Thus, the present invention also provides:
- A heparin derivative as defined herein, or a pharmaceutically acceptable salt thereof, for use in the inhibition, prevention and/or treatment of tumour angiogenesis.
- A method of inhibiting, preventing and/or treating tumour angiogenesis, the method comprising administering to a subject in need of such treatment a therapeutically effective amount of a heparin derivative as defined herein, or a pharmaceutically acceptable salt thereof.
- The use of a heparin derivative as defined herein, or a pharmaceutically acceptable salt thereof, in the manufacture of a medicament for use in the inhibition, prevention and/or treatment of tumour angiogenesis.

### Cancer metastasis

The heparin derivatives of the present invention provide a means of preventing and/or treating cancer metastasis by inhibiting the activity of circulating galectin-3 in the bloodstream.

Thus, the present invention further provides:
- A heparin derivative as defined herein, or a pharmaceutically acceptable salt thereof, for use in the prevention and/or treatment of cancer metastasis.
- A method of preventing and/or treating cancer metastasis, the method comprising administering to a subject in need of such treatment a therapeutically effective amount of a heparin derivative as defined herein, or a pharmaceutically acceptable salt thereof.
- The use of a heparin derivative as defined herein, or a pharmaceutically acceptable salt thereof, in the manufacture of a medicament for use in the prevention and/or treatment of cancer metastasis.

### Combination Therapies

The anti-cancer treatments defined hereinbefore may be applied as a sole therapy or may involve, in addition to the heparin derivative of the invention, conventional surgery or radiotherapy or chemotherapy.

Such chemotherapy may include one or more of the following categories of anti-tumour agents:-
(i) other antiproliferative/antineoplastic drugs and combinations thereof, as used in medical oncology, such as alkylating agents (for example cis-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);
(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) anti-invasion agents [for example c-Src kinase family inhibitors like 4-(6-chloro-2,3-methylenedioxyanilino)-7-[2-(4-methylpiperazin-1-yl)ethoxy]-5-tetrahydropyran-4-yloxyquinazoline (AZD0530; International Patent Application WO 01/94341), N-(2-chloro-6-methylphenyl)-2-{6-[4-(2-hydroxyethyl)piperazin-1-yl]-2-methylpyrimidin-4-ylamino}thiazole-5-carboxamide (dasatinib, BMS-354825; J. Med. Chem., 2004, 47, 6658-6661) and bosutinib (SKI-606), and metalloproteinase inhibitors like marimastat, inhibitors of urokinase plasminogen activator receptor function or antibodies to Heparanase];
(iv) inhibitors of growth factor function: for example such inhibitors include growth factor antibodies and growth factor receptor antibodies (for example the anti-erbB2 antibody trastuzumab [Herceptin™], the anti-EGFR antibody panitumumab, the anti-erbB1 antibody cetuximab [Erbitux, C225] and any growth factor or growth factor receptor antibodies disclosed by Stern et al. Critical reviews in oncology/haematology, 2005, Vol. 54, pp11-29); such inhibitors also include tyrosine kinase inhibitors, for example inhibitors of the epidermal growth factor family (for example EGFR family tyrosine kinase inhibitors such as *N*-(3-chloro-4-fluorophenyl)-7-methoxy-6-(3-morpholinopropoxy)quinazolin-4-amine (gefitinib, ZD1839), N-(3-ethynylphenyl)-6,7-bis(2-methoxyethoxy)quinazolin-4-amine (erlotinib, OSI-774) and 6-acrylamido-*N*-(3-chloro-4-fluorophenyl)-7-(3-morpholinopropoxy)-quinazolin-4-amine (CI 1033), erbB2 tyrosine kinase inhibitors such as lapatinib); inhibitors of the hepatocyte growth factor family; inhibitors of the insulin growth factor family; inhibitors of the platelet-derived growth factor family such as imatinib and/or nilotinib (AMN107); inhibitors of serine/threonine kinases (for example Ras/Raf signalling inhibitors such as farnesyl transferase inhibitors, for example sorafenib (BAY 43-9006), tipifarnib (R115777) and lonafarnib (SCH66336)), inhibitors of cell signalling through MEK and/or AKT kinases, c-kit inhibitors, abl kinase inhibitors, PI3 kinase inhibitors, Plt3 kinase inhibitors, CSF-1R kinase inhibitors, IGF receptor (insulin-like growth factor) kinase inhibitors; aurora kinase inhibitors (for example AZD1152, PH739358, VX-680, MLN8054, R763, MP235, MP529, VX-528 AND AX39459) and cyclin dependent kinase inhibitors such as CDK2 and/or CDK4 inhibitors;
(v) antiangiogenic agents such as those which inhibit the effects of vascular endothelial growth factor, [for example the anti-vascular endothelial cell growth factor antibody bevacizumab (Avastin™) and for example, a VEGF receptor tyrosine kinase inhibitor such as vandetanib (ZD6474), vatalanib (PTK787), sunitinib (SU11248), axitinib (AG-013736), pazopanib (GW 786034) and 4-(4-fluoro-2-methylindol-5-yloxy)-6-methoxy-7-(3-pyrrolidin-1-ylpropoxy)quinazoline (AZD2171; Example 240 within WO 00/47212), compounds such as those disclosed in International Patent Applications WO97/22596, WO 97/30035, WO 97/32856 and WO 98/13354 and compounds that work by other mechanisms (for example linomide, inhibitors of integrin αvβ3 function and angiostatin)];
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) an endothelin receptor antagonist, for example zibotentan (ZD4054) or atrasentan;
(viii) antisense therapies, for example those which are directed to the targets listed above, such as ISIS 2503, an anti-ras antisense;
(ix) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(x) immunotherapy approaches, including for example *ex-vivo* and *in-vivo* approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

The anti-metastatic treatments defined hereinbefore may also be applied as a sole therapy or may involve, in addition to the heparin derivative of the invention, treatment with surgery, radiotherapy, chemotherapy or another anti-metastatic agent.

Such conjoint treatment may be achieved by way of the simultaneous, sequential or separate dosing of the individual components of the treatment. Such combination products employ the heparin derivatives of this invention within the dosage range described hereinbefore and the other pharmaceutically-active agent within its approved dosage range.

According to this aspect of the invention there is provided a combination suitable for use in the treatment of a cancer and/or cancer metastasis comprising a heparin derivative of the invention as defined hereinbefore, or a pharmaceutically acceptable salt thereof, and another anti-tumour agent and/or anti-metastatic agent.

In a further aspect of the invention there is provided a heparin derivative of the invention or a pharmaceutically acceptable salt thereof, for use in the treatment of cancer and/or cancer metastasis, wherein the heparin derivative is administered in combination with an anti-tumour agent or anti-metastatic agent.

Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the beneficial effect of the combination.

According to a further aspect of the invention there is provided a pharmaceutical composition which comprises a heparin derivative of the invention, or a pharmaceutically acceptable salt thereof, in combination with another anti-tumour or anti-metastatic agent.

### Examples

The invention will now be illustrated in the following Examples.

### Example 1 - Preparation of Modified Heparin Derivatives

Chemically modified heparin compounds (A) to (I) were prepared by the following combinations of reactions (a) to (g) set out below:
(A) PIMH starting material (Celsus Labs, Cincinnati, OH);
(B) *N*-acetyl heparin (d) (f);
(C) Ido 2-de-*O*-sulphated heparin (a);
(D) 6-*O*-desulphated heparin (b) (e);
(E) Ido 2-de-*O*-sulphated, *N*-acetylated heparin (a) (d) (f);
(F) 6-*O*-desulphated, *N*-acetylated heparin (b) (f);
(G) 6-*O*-desulphated, 2-*O*-desulphated heparin (c) (e);
(H) 6-*O*-desulphated, 2-*O*-desulphated, *N*-acetylated heparin (c) (f); and
(I) Per-sulphated heparin (g) (e).

Compounds were characterized by ¹H and ¹³C NMR as previously described. (Yates et al., Carbohydrate Research 1996, 294, 15-27.) Compounds were desalted, lyophilized and re-suspended in the appropriate buffer prior to assay.

### Chemical Reactions

(a) Selective removal of iduronate 2-*O*-sulphate was achieved as described by Jaseja and Perlin. (Jaseja, M.; Rej, R. N.; Sauriol, F.; Perlin, A. S. Can. J. Chem. 1989, 67, 1449-1456.) Note that there is concomitant modification in the small number of *N-* and 3-0-sulphated glucosamine units. (Santini, F.; Bisio, A.; Guerrini, M.; Yates, E. A. Carbohydrate Research 1997, 302, 103-108.)
(b) Selective removal of glucosamine 6-*O*-sulphate was carried out according to a modification (Yates, E. A. et al. *supra*.) of the method described. (Inoue, S.; Miyawaki, M. Analytical Biochemistry 1975, 65, 164-174.)
(c) Complete removal of *O*- and *N*-sulphates was achieved using solvolytic de-sulphation by the method described. (Inoue, S.; Miyawaki, M. *supra*.)
(d) Selective de-*N*-sulphation was carried out employing controlled solvolytic de-sulphation under kinetic control as described. (Inoue, Y.; Nagasawa, K. Carbohydrate Research 1976, 46, 87-95.)
(e) Re *N*-sulphation was achieved by use of trimethylamine.sulfur trioxide complex as described. (Lloyd, A. G.; Embery, G.; Fowler, L. J. Biochemical Pharmacology 1971, 20, 637-648.)
(f) Re *N*-acetylation employed acetic anhydride in saturated sodium bicarbonate. (Yates, E. A. et al. *supra*.)
(g) Complete *O*-sulphation of all available hydroxyl groups was carried out using excess sulfur trioxide pyridine complex on the tetrabutylammonium salt of heparin in pyridine as described (Yates, E. A.; Santini, F.; De Cristofano, B.; Payre, N.; Cosentino, C. et al. Carbohydrate Research 2000, 329, 239-247.) followed by re-*N*-sulphation (Lloyd, A. G. et al. *supra*.) taking precautions to avoid formation of an unusual *N*-sulfoaziridine modification. (Yates, E. A.; Santini, F.; Bisio, A.; Cosentino, C. Carbohydrate Research 1997, 298, 335-340)

### Compound purity

The starting material for all chemical modifications was porcine intestinal mucosal heparin (Celsus Labs, Cincinnati, OH, USA; lot PH-42800 with anticoagulant activity 201 IU/mg).

Each polysaccharide **A**-**H** was subjected to purification by size-exclusion chromatography (Sephadex G-25, recovering only the exclusion limit; M_{w}> 5 KDa) and treated with ion exchange resin (Dowex, W-50, Na⁺ form) prior to NMR and activity testing.

Size-exclusion chromatography analysis of the 8 polysaccharides was also conducted with a TSK gel G2000SW_{XL} column (7.8 mm x 30 cm with 0.5 µm particle size; Supelco) eluting with water at 1 ml/min and detecting at 190 nm. All the samples exhibited a single major peak, with very similar retention times (mean, 6.07 minutes; σₙ₋₁=0.05). In all cases, there were no contaminants greater than 5 %.

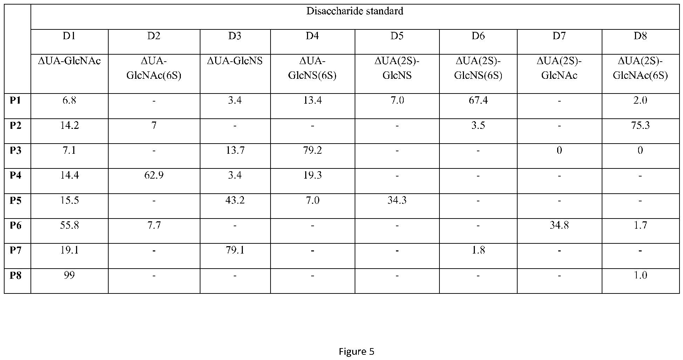

Polysaccharides **A-H** were also exhaustively digested with a mixture of heparitinases I, II and III to their constituent disaccharides (here denoted D1 to D8) determined. Disaccharides were separated by strong-anion exchange HPLC (Propac PA-1 column, Dionex UK; [ref 2]) and quantified (A₂₃₂) with reference to authentic standards (Dextra Labs, Reading, UK) and showed the following composition (%). In all cases, unidentified peaks were < 5% of the total area of the constituents. The data is shown in Figure 5 and conforms exactly to the compositions predicted from the modifications performed, and are in agreement with the NMR structure data (see below).

In Figure 5, P1=A, standard heparin; P2=B, *N*-acetylated; P3=C, 2-de-*O*-sulfated; P4=E: 2-de-*O*-sulfated and *N*-acetylated; P5=D, 6-de-*O*-sulfated; P6=F, 6-de-O-sulfated and *N-*acetylated; P7=G, 2-,6-de-*O*-sulfated and *N*-sulfated; P8=H, 2-,6-de-*O*-sulfated and *N*-acetylated.

### NMR Spectroscopy

The polysascharides were characterised by ¹H and ¹³C NMR to confirm their structure [Yates, E. A.; Santini, F.; Bisio, A.; Cosentino, C. Carbohydrate Research 1997, 298, 335-340]. NMR spectra were recorded in D₂O at 40 °C on a 400 MHz instrument. Assignment was by a combination of COSY, TOCSY, HMBC two-dimensional spectra. ¹³C spectra were recorded on 150 mg samples of the polysaccharide. Chemical shift values were recorded relative to trimethylsilyl propionate as reference standard at 40 °C.

**Table of ¹H and ¹³C NMR chemical shift values for heparin derived polysaccharides A-H**

| | Glucosamine | | | | | | | Iduronate | | | | |
|---|---|---|---|---|---|---|---|---|---|---|---|---|
| Polysaccharide | A-1 | A-2 | A-3 | A-4 | A-5 | A-6 | | I-1 | I-2 | I-3 | I-4 | I-5 |
| **A** | 99.5 | 60.7 | 72.5 | 78.8 | 72.0 | 69.2 | | 102.1 | 78.9 | 72.1 | 79.0 | 72.3 |
| | 5.42 | 3.31 | 3.69 | 3.79 | 4.05 | 4.30-4.42 | | 5.23 | 4.37 | 4.22 | 4.14 | 4.82 |
| | | | | | | | | | | | | |
| **B** | 96.6 | 56.2 | 73.0 | 79.3 | 72.3 | 69.6 | | 102.2 | 76.8 | 67.3 | 74.2 | 70.8 |
| | 5.15 | 4.03 | 3.76 | 3.78 | 4.04 | 4.31-4.37 | | 5.20 | 4.37 | 4.31 | 4.08 | 4.91 |
| | | | | | | | | | | | | |
| **C** | 98.1 | 60.3 | 72.4 | 80.1 | 71.5 | 68.7 | | 104.6 | 71.1 | 70.4 | 77.2 | 71.2 |
| | 5.34 | 3.24 | 3.65 | 3.71 | 4.02 | 4.36 4.23 | | 5.04 | 3.78 | 4.12 | 4.08 | 4.84 |
| | | | | | | | | | | | | |
| **D** | 100.0 | 60.8 | 72.4 | 80.5 | 73.8 | 62.6 | | 102.0 | 77.6 | 70.7 | 78.7 | 71.4 |
| | 5.31 | 3.27 | 3.71 | 3.70 | 3.89 | 3.86-3.88 | | 5.26 | 4.35 | 4.25 | 4.06 | 4.84 |
| | | | | | | | | | | | | |
| **E** | 97.1 | 56.2 | 72.5 | 79.6 | 71.8 | 68.8 | | 104.6 | 72.0 | 71.4 | 77.0 | 71.9 |
| | 5.18 | 4.00 | 3.78 | 3.79 | 4.08 | 4.37-4.26 | | 5.01 | 3.75 | 3.42 | 4.10 | 4.78 |
| | | | | | | | | | | | | |
| | | | | | | | | | | | | |
| **F** | 96.8 | 56.6 | 72.9 | 80.6 | 74.2 | 62.9 | | 102.3 | 76.6 | 67.1 | 74.1 | 70.6 |
| | 5.14 | 4.03 | 3.79 | 3.76 | 3.91 | 3.87-3.92 | | 5.26 | 4.37 | 4.28 | 4.07 | 4.91 |
| | | | | | | | | | | | | |
| **G** | 98.2 | 60.5 | 72.5 | 80.2 | 73.5 | 62.4 | | 104.3 | 72.2 | 71.5 | 77.8 | 72.2 |
| | 5.39 | 3.26 | 3.67 | 3.72 | 3.87 | 3.84-3.88 | | 4.95 | 3.74 | 4.11 | 4.08 | 4.77 |
| | | | | | | | | | | | | |
| **H** | 97.1 | 56.2 | 72.3 | 79.6 | 73.7 | 62.3 | | 104.3 | 72.5 | 72.2 | 77.3 | 72.6 |
| | 5.18 | 3.97 | 3.76 | 3.74 | 3.89 | 3.85-3.88 | | 4.92 | 3.69 | 3.89 | 4.07 | 4.73 |
| | | | | | | | | | | | | |
| **I** | 99.6 | 59.3 | 82.9 | 76.8 | 72.1 | 68.7 | | 100.8 | 73.6 | 72.9 | 73.3 | 69.8 |
| | 5.32 | 3.50 | 4.48 | 4.04 | 4.05 | 4.27-4.41 | | 5.32 | 4.55 | 4.72 | 4.39 | 5.05 |

The ¹H chemical shift values quoted for position-6 of glucosamine residues (A-6) are intervals. Signals from the carbonyl group of iduronate and acetyl CH₃ groups of *N*-acetylated glucosamine derivatives are not shown.

### Example 2 - ELISA assessment of the heparin derivatives described in Example 1 on galectin-3 binding to asialo bovine mucin

### Experimental protocol:

Half-volume 96-well plates were coated with 10µg/ml of asialo bovine mucin (ASM), Antarctic fish (*Trematomus borchgrevinki)* anti-freeze glycoprotein or asialo fetuin in coating buffer (1.6g Na₂CO₃, 1.46g NaHCO₃ in 1L, pH9.6) overnight at 4°C. After 2 washes with washing buffer (0.05% Tween 20 in PBS), the plates were applied with 50µl/well blocking buffer (1% bovine serum albumin, BSA, in PBS) for 1 hr at room temperature. The plates were washed with washing buffer. Recombinant galectin-3 (1µg/ml) was pre-incubated with 25µg/ml BSA or various concentrations of the heparin derivatives described in Example 1 (0 µg/ml, 1 µg/ml, 5 µg/ml or 20 µg/ml) for 30 mins at room temperature before application to the coated wells for 2 hrs at room temperature. The wells were washed twice with washing buffer before application of biotinylated anti-galectin-3 antibody (1.5µg/ml in blocking buffer) for 1hr at room temperature. The plates were washed with washing buffer and applied with 50 µl/well ExtrAvidin-HRP (1:10,000 dilution in blocking buffer) for 1hr. The wells were washed twice with washing buffer before developed with SigmaFAST OPD. The reaction was stopped with 25µl 4M sulphuric acid and the plates were read at 492nm by a microplate reader.

### Results:

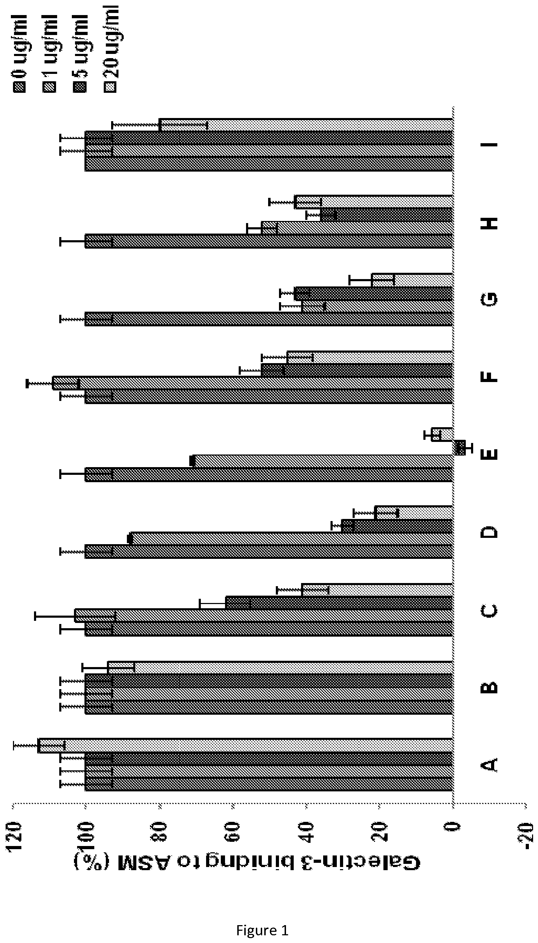

Figure 1 shows the effects of the heparin derivatives described in Example 1 herein on galectin-3 binding to TF-expressing asialo bovine mucin (A, standard heparin; B, *N*-acetylated; C, 2-de-*O*-sulfated; D, 6-de-*O*-sulfated; E: 2-de-*O*-sulfated and *N*-acetylated; F, 6-de-O-sulfated and *N*-acetylated; G, 2-,6-de-*O*-sulfated and *N*-sulfated; H, 2-,6-de-*O*-sulfated and *N*-acetylated; I, over -sulfated). It can be seen that the six 2 or 6-de-O-sulfated heparin derivatives (C to H - i.e. the heparin derivatives according to the present invention), produced by chemical modification of porcine heparin, show significant inhibition of galectin-3 binding to TF-expressing asialo bovine mucin (ASM) in the galectin-3 ELISA assay. Over-sulphation of a heparin derivative (I) reverses this inhibitory activity.

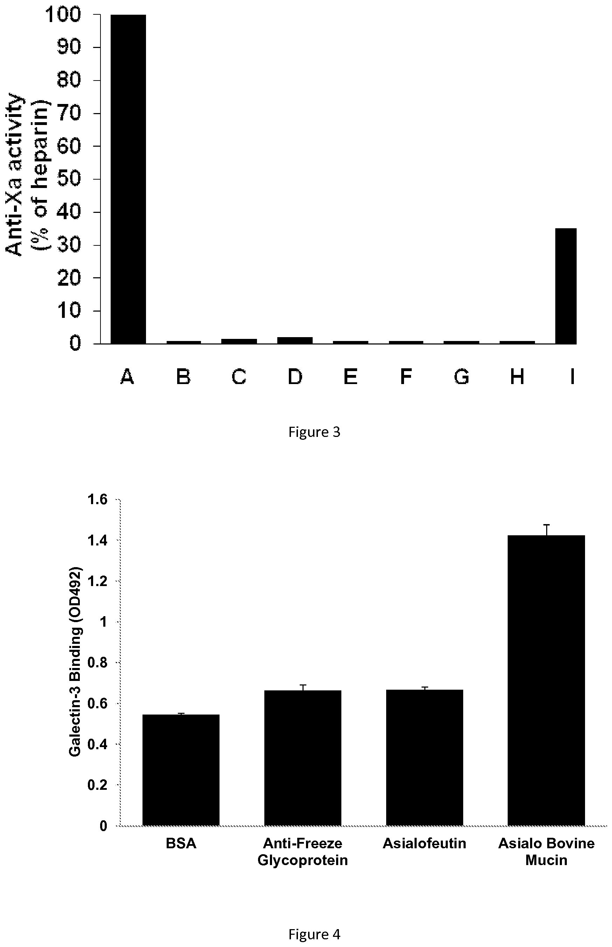

Figure 4 shows the binding of galectin-3 to BSA, anti-freeze glycoprotein, asialofeutin, and asialo bovine mucin. Asialo bovine mucin shows the greatest binding.

### Example 3 - Assessment of the effect of the heparin-derivatives of Example 1 on galectin-3-mediated cancer cell adhesion to endothelial cells

### Experimental protocol:

Human microvascular lung endothelial cells (HMVEC-Ls) were seeded into 96-well cell plates at 5.0x10⁴ cells/ml in EBM-2 culture medium in a humidified atmosphere of 5% CO₂, 95% air, for 2-3 days at 37°C until a complete monolayer was formed.

Human colon cancer HT29-5F7 cells were released from culture flasks with Non-enzymatic Cell Dissociation Solution. The cells were washed 3 times with PBS, re-suspended in serum-free DMEM at 1x10⁵ cells/ml and incubated with 10µl/ml Calcein AM at 37°C for 30 min on a shaking waterbed at 80rpm. The cells were washed twice with serum free DMEM and re-suspended in fresh serum-free DMEM at 5x10⁴/ml.

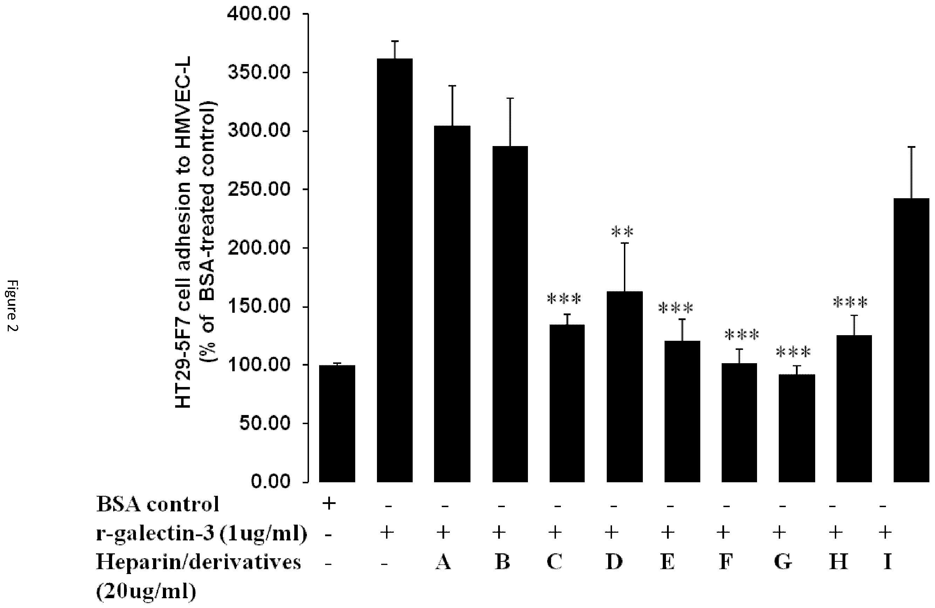

Recombinant galectin-3 (1µg/ml) was pre-incubated with 25µg/ml BSA or 20µg/ml heparin derivative described in Example 1 for 30 min before mixed with the cancer cell suspension for 1 hr at 37°C. The cell suspension was applied to the HMVEC-L monolayers at 37°C for 1 hr (Fig 2). The endothelial monolayers were washed with PBS before the endothelial cell-associated fluorescence was obtained by a fluorescence micro-plate reader (490nm excitation and 520 nm emission).

### Results:

Figure 2 shows the effects of the heparin derivatives described in Example 1 herein on the inhibition of galectin-3-mediated adhesion of human colon cancer HT29-5F7 cells to monolayer of human micro-vascular lung endothelial cells (HMVEC-Ls) in culture. The six de-O-sulfated heparin derivatives of the present invention (C to H) all cause a marked reduction of galectin-3-mediated adhesion of human colon cancer HT29-5F7 cells to monolayer of human micro-vascular lung endothelial cells (HMVEC-Ls) in culture. The heparin derivatives that best inhibit galectin-3 - induced cancer cell adhesion to endothelial cells are, fortuitously, those that have little or no anti-coagulant effect (see Example 4).

### Example 4 - The anticoagulant activity of the heparin derivatives prepared in Example 1

### Experimental protocol:

The anti-factor Xa activity was measured against a porcine mucosal heparin (PIMH) standard of known activity using a diagnostic grade Coatest Heparin test kit (Chromogenix, MA), adapted to a 96-well plate format, reading A405.

### Results:

Figure 3 and the Table below show the anticoagulant activity (anti-Xa activity) of the heparin derivatives described in Example 1 herein. Seven of the heparin-derivatives (B to H) including the six O-de-sulphated heparins of the present invention (C to H) show <1.5% anti-Xa activity compared with that of the standard heparin (A). The heparin derivatives that best inhibit galectin-3-induced cancer cell adhesion to endothelial cells (Example 3) have little or no anti-coagulant effect.

**Anticoagulant activity data [Patey et al. J Med Chem. 2006, 49, 6129-6132]**

| **Compound** | | **R₁** | **R₂** | **R₃** | **Anti-coagulant activity** |
|---|---|---|---|---|---|
| (A) | PMIH | SO₃ | SO₃ | SO₃ | 100% |
| (B) | N-acetyl | SO₃ | SO₃ | COCH₃ | 0.03% |
| (C) | UA-2-OH | H | SO₃ | SO₃ | 0.4% |
| (D) | GlcN-6-OH | SO₃ | H | SO₃ | 0.5% |
| (E) | UA-2-OH, N-Acetyl | H | SO₃ | COCH₃ | 0.03% |
| (F) | GlcN-6-OH, N-acetyl | SO₃ | H | COCH₃ | 0.03% |
| (G) | UA-2-OH, GlcN-6-OH | H | H | SO₃ | 0.03% |
| (H) | UA-2-OH, GlcN-6-OH, N-acetyl | H | H | COCH₃ | 0.03% |
| (I) | Per-sulfated | SO₃ | SO₃ | SO₃ | 35.0% |

### Example 5 - Preparation of polysaccharide fragments

Heparin fragments varying in size were prepared by the partial digestion and size separation of the modified heparins (C) to (H) by the procedure outlined below.

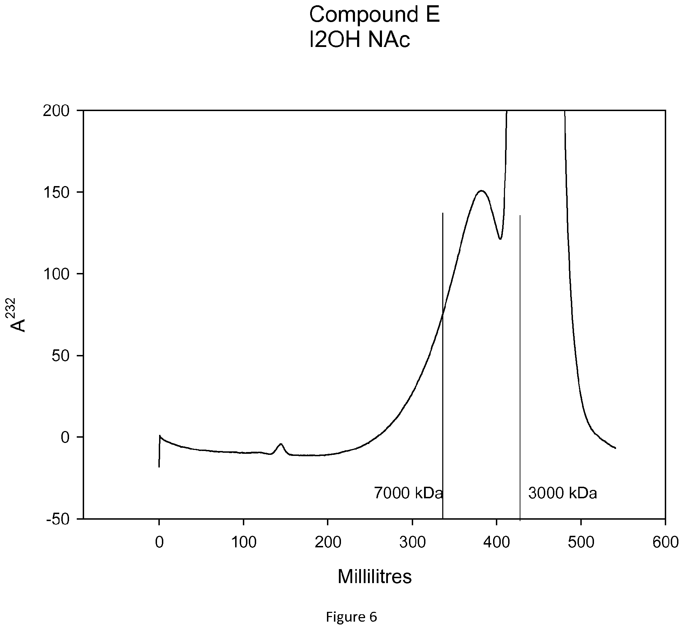

50-100 mg of each modified heparin was subjected to free radical digestion for 24 hours at room temperature using free radical digestion as previously described (Liu and Perlin, 1994). Digested saccharides were separated using a GE Healthcare XK26/100 Sephadex G100 column (bed volume 450 ml) running isocratically with 500 mM ammonium bicarbonate (Sigma). A sample profile for the digestion of the modified heparin (E) is presented in Figure 6.

Fractions (2 ml) were collected and pooled into >7000 Da, 3000-7000 Da and <3000 Da fractions. Pooled fractions were freeze dried repeatedly to remove ammonium bicarbonate and weighed.

In the following discussion and the accompanying figures:
- fragments C1, D1, E1, F1, G1 and H1 (prepared from modified heparins C, D, E, F, G and H respectively) are all greater than 7000 Da in size;
- fragments C2, D2, E2, F2, G2 and H2 (prepared from modified heparins C, D, E, F, G and H respectively) are all 3000-7000 Da in size, and
- fragments C3, D3, E3, F3, G3 and H3 (prepared from modified heparins C, D, E, F, G and H respectively) are all less than 3000 Da in size.

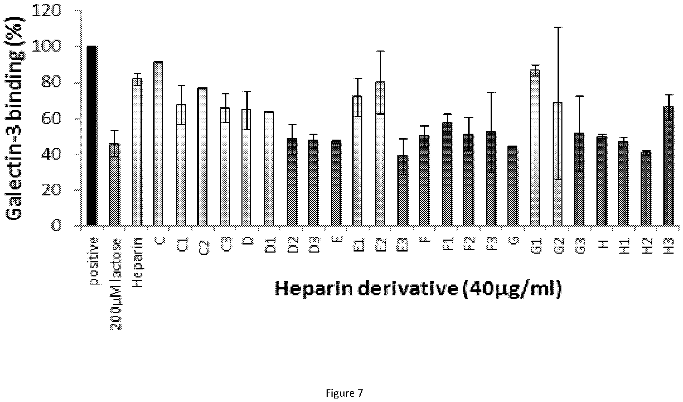

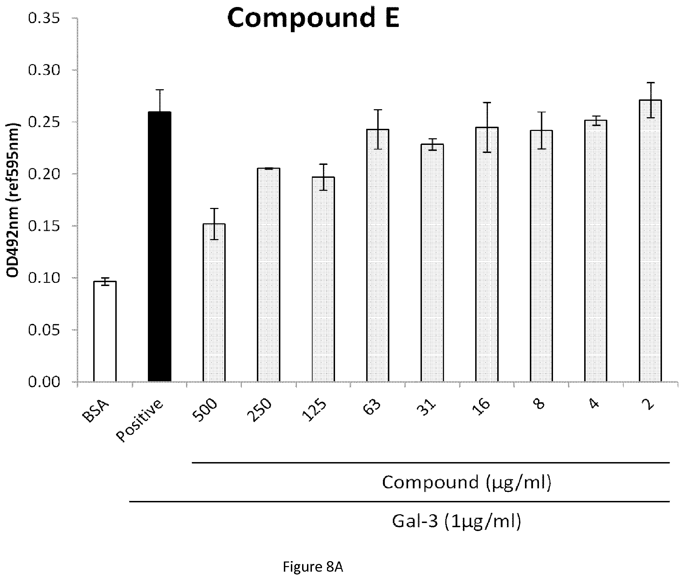

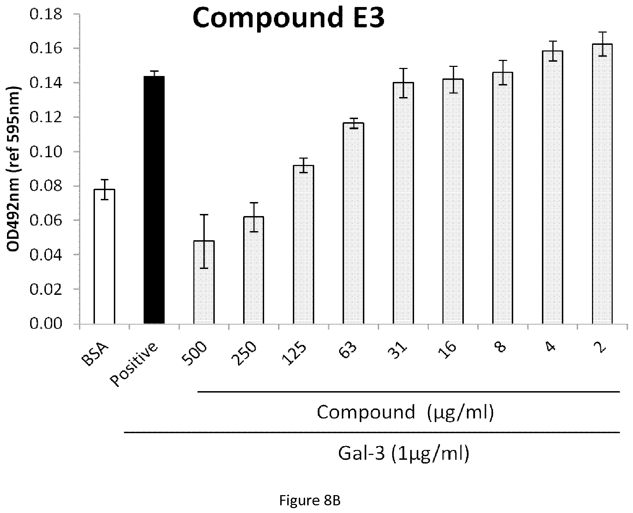

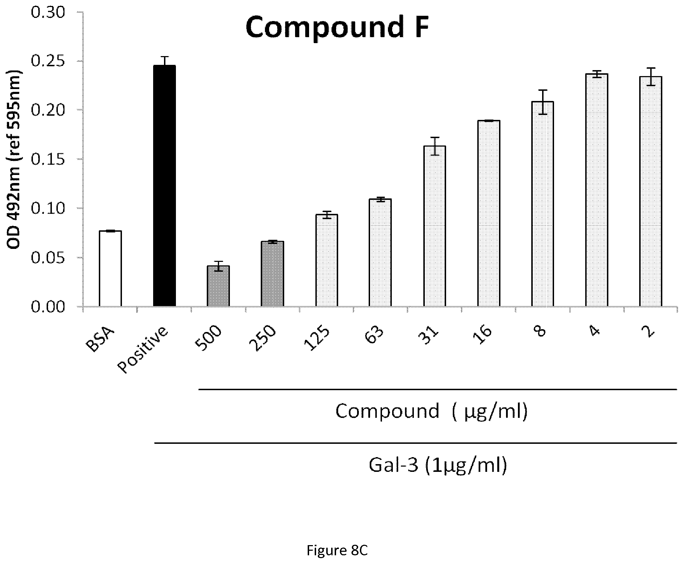

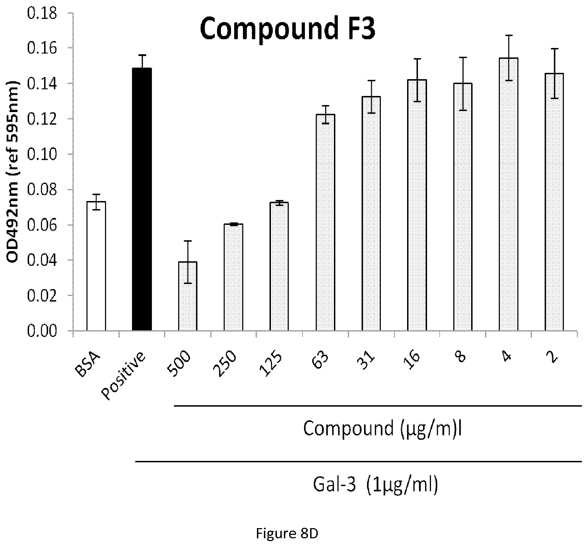

### Example 6 - Assessments of the heparin derivatives and fragments on galectin-3 binding to TF-expressing glycan asialo fetuin by ELISA (Figures 7 and 8A-D).

High-Binding, Half-Volume 96-well plates were coated with 50µl/well of control BSA or 5µg/ml TF-expressing glycan asialo fetuin (ASF) in coating buffer (1.6g Na2CO3, 1.46 NaHCO3 in 1L H₂O, pH 9.6) overnight at room temperature. The plates were washed twice with 80µl/well washing buffer (0.05% Tween-20 in PBS) before 80µl/well blocking buffer (1% BSA in PBS) was introduced for 30 min at room temperature to block the non-specific binding. One microgram/ml recombinant galectin-3 was incubated with various concentrations of the heparin derivatives or lactose for 30 minutes at room temperature before introduced to the coated plates at room temperature for 1 hr. The plates were washed twice with 80µl/well of washing buffer before addition of 50µl/well of anti-galectin-3 antibody (1µg/ml in PBS) for 1 hr at room temperature. After washing with 80µl/well washing buffer, 50µl/well HRP-anti-mouse antibody (1:10,000 dilution in PBS) were introduced for 1 hr at room temperature. The plates were washed twice with 80µl/well washing buffer. SigmaFAST OPD dual tablets were dissolved into 20ml of distilled H₂0 and 50µl/well was added the plates until a yellow colour developed (approximately 20 minutes). The plates were then read by a microplate reader at 492nm with a reference of 595nm.

### Results and Conclusion:

A number of heparin derivatives including D2, D3, E, E3, F, F1, F2, F3, G, H, H1, H2 and H3 at 40µg/ml inhibit galectin-3 (1µg/ml) binding to TF-expressing glycan asialo fetuin (Figure 7) when assessed by ELISA.

Heprin derivatives E, E3, F and F3 at 2 -500µg/ml produce a dose-dependent inhibition of galectin-3 (1µg/ml) binding to asialo fetuin (Figures 8A-D).

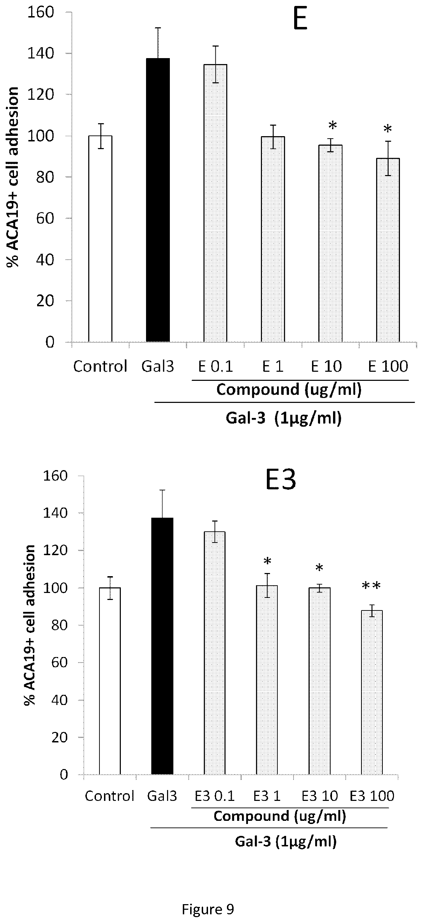

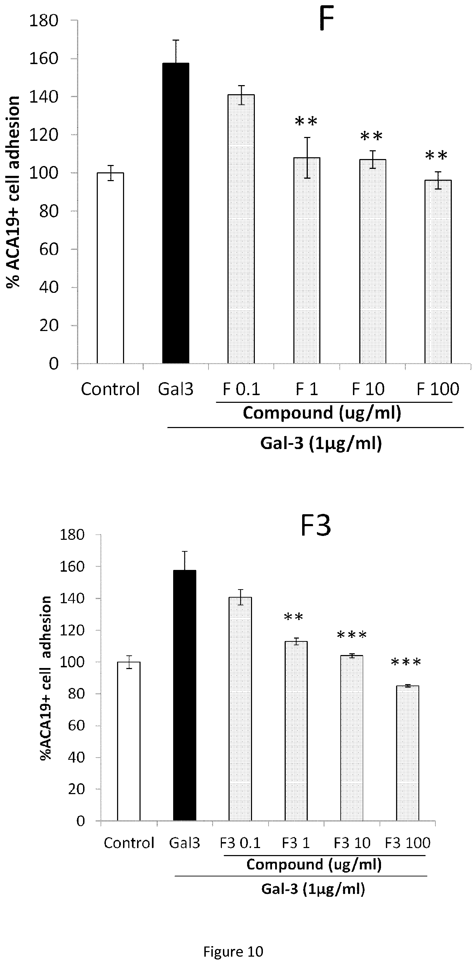

### Example 7 - Assessments of the heparin derivatives on galectin-3-mediated adhesion of ACA19+ cancer cells to endothelial monolayer (Figures 9 and 10).

The MUC1 positive transfectants (ACA19+) of human melanoma cells were released from the culture flasks with the non-enzymatic cell dissociation solution (NECDS). The cells were washed twice with serum-free DMEM (containing 0.5mg/ml BSA) and incubated/labelled with 10µl Calcein AM cell labelling solution at 37°C for 30 min. The cells were washed twice with serum-free DMEM and re-suspended to 2x10⁵ cells/ml with serum-free DMEM. Human umbilical vein endothelial cells (HUVECs) were cultured at 37°C for 2 days in EBM-2 endothelial culture medium for the formation of endothelial monolayer. The endothelial culture medium was removed and the cells were washed and introduced with serum-free DMEM. Various concentrations of the heparin derivatives were added to the HUVEC suspension with or without immediate introduction of 1 µg/ml galectin-3. After addition of 100µl ACA19+ cell suspension for 30-45 min at 37°C, the endothelial monolayer was washed twice with serum-free DMEM to remove unbound ACA19+ cells, lysed with 100µl 0.25% SDS and applied to reading by a fluorescence microplate reader.

### Results and Conclusion:

Heparin derivatives E, E3, F and F3 at 1 to 100µg/ml show a dose-dependent inhibition of galectin-3 (1µg/ml)-induced adhesion of human melanoma ACA19+ cells to HUVEC monolayer (Figures 9 and 10).

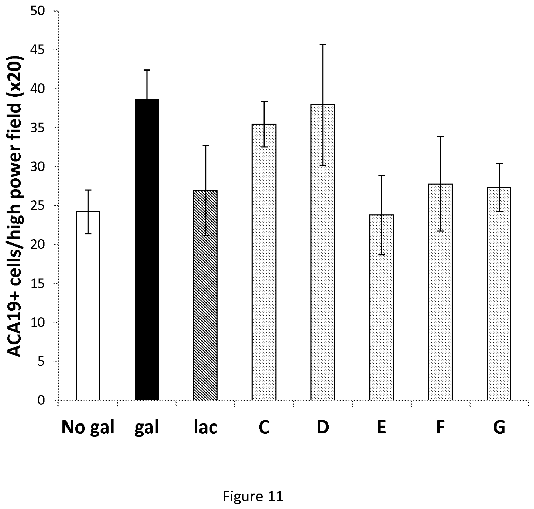

### Example 8 - Assessments of the heparin derivatives on galectin-3-mediated invasion of ACA19+ cancer cells through endothelial monolayer (Figures 11, 12 and 13)

HUVECs were cultured in 24-well plate transwell inserts (with 8-µm pore filters, BD Falcon) for 2-3 days to allow tight formation of cell monolayer. Monolayer integrity was monitored by measuring trans-endothelial electrical resistance (TEER) using a Volt Ohm Meter and monolayer with TEER > 300Ω/cm² were used for trans-endothelial assessment. ACA19+ cells, released by NECDS, labelled with 10µl calcein AM cell labelling solution and suspended to 1x10⁵ cells/ml with serum-free DMEM. Recombinant galectin-3 (1µg/ml) was incubated with or without various concentrations of heparin derivatives or lactose for 30 min at 37°C before addition to the ACA19+ cell suspension for 1 hr at 37°C in a shaking waterbath (80rpm). The culture medium in the trans-wells was removed and 400µl ACA19+ cell suspension was introduced to the HUVEC monolayer. After 1 hr incubation at 37°C, the non-adherent cancer cells were removed by two washes with medium and the adhesion cells-HUVEC monolayer were incubated at 37°C for 24 hr before the cells were fixed with 2% paraformaldehyde. The cells at the upper side of the transwell membrane were removed with a cotton swab and fluorescent cells migrated to the bottom side of the transwell membrane were counted using an Olympus B51 fluorescence microscope with a x20 objective.

### Results and Conclusion:

Heparin derivatives E, F and G, but not C and D, at 100µg/ml inhibit galectin-3-mediated trans-endothelial invasion of human melanoma ACA19+ cells through HUVEC monolayer (Figure 11).

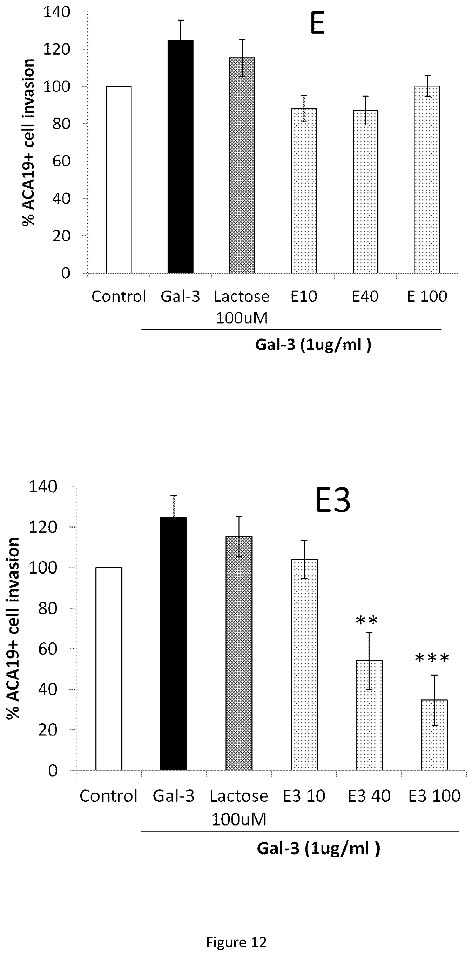

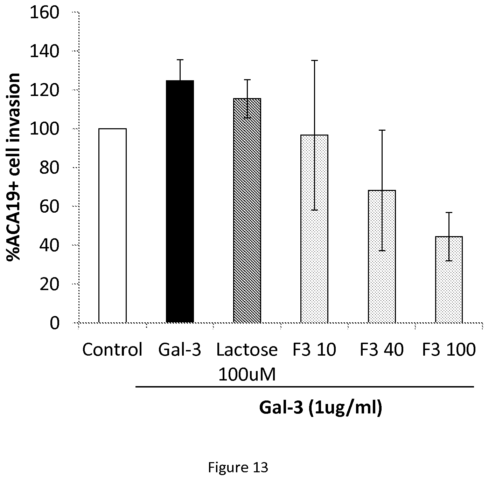

Heprin derivatives E3 and F3 at 10 -100µg/ml inhibit galectin-3 (1µg/ml)-mediated trans-endothelial invasion of human melanoma ACA19+ cells through HUVEC monolayer (Figure 12 and 13).

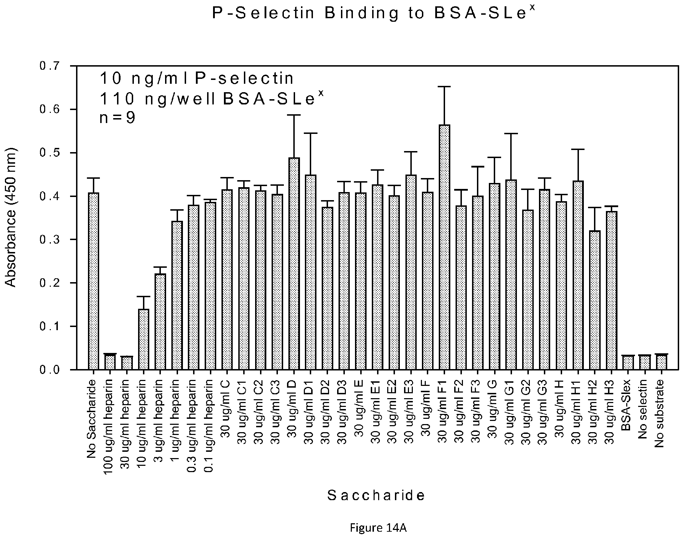

### Example 9 - Assessments of the heparin derivatives on binding of selectins to their ligands (Figures 14A-C and Table 3)

The ability of the saccharides to interfere with selectin binding to sialyl-Lewis X antigen was tested using an ELISA assay as previously described (Nelson et al, 1993). Sialyl Lewis X antigen conjugated to BSA (Dextra Labs) was coated onto an ELISA plate (110 ng/well in 50 mM sodium bicarbonate pH 9.5) for 1 hour at room temperature and blocked with blocking buffer (20 mM HEPES, pH 7.4,150 nM NaCl, 2 mM CaCl₂ and 20 mg/nl BSA) for a further hour at room temperature. E-selectin- (10 ng/ml), P-selectin- (10 ng/ml) or L-selectin- (30 ng/ml) Fc conjugates (All from R&D systems) were incubated with saccharides (up to 100 ug/ml) or heparin for 1 hour at room temperature in binding buffer then added to the sialyl Lewis X and incubated for 3 hours at room temperature. Unbound selectin was washed away with binding buffer and bound selectin was quantified using an HRP-conjugated anti-Fc antibody (Pierce) followed detection by HRP Substrate Solution (R&D Systems) and quantification at 450 nm Heparin reduced L-selectin and P-selectin binding to sialyl Lewis X with IC50 values of 1.63 ug/ml and 4 ug/ml respectively (Table 1). Heparin did not compete for E-selectin binding to sialyl Lewis X. None of the saccharides caused reduction in binding of selectin to sialyl Lewis X up to 100 ug/ml.

### Results and Conclusions:

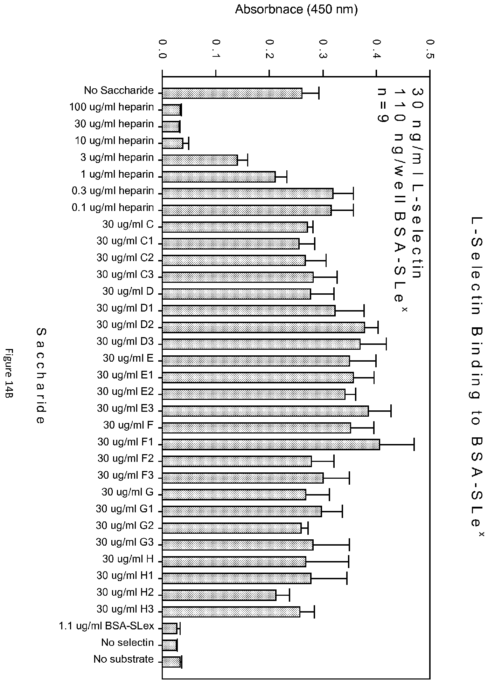

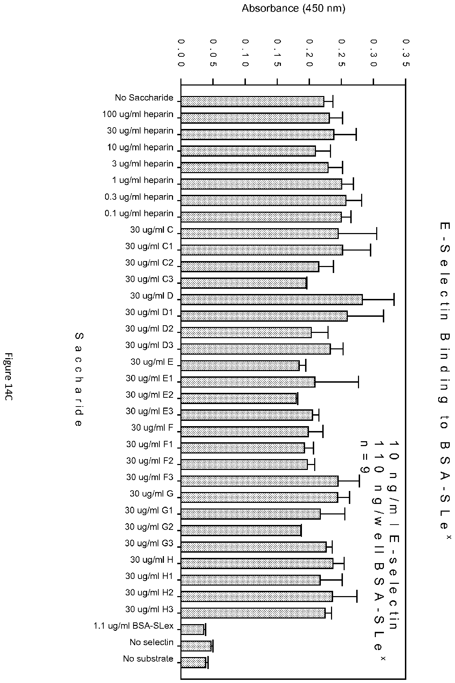

None of the heparin derivatives, fractionated or non-fractionated, including E, E3, F and F3 shows inhibition to binding of P-, L- or E-selectins to their ligand Sle^{X} (Figures 14A, 14B and 14C; Table 3).

**Table 3: Inhibition of selectin binding to sialyl Lewis X by heparin and polysaccharide fractions**

| Compound | Structure | IC50 L-selectin | IC50 P-selectin | IC50 E-selectin |
|---|---|---|---|---|
| Heparin | | 1.63 | 4.0 | NI |
| C | I2OH | NI | NI | NI |
| C1 | I2OH >7000 | NI | NI | NI |
| C2 | I2OH 7000-3000 | NI | NI | NI |
| C3 | I2OH <3000 | NI | NI | NI |
| D | A6OH | NI | NI | NI |
| D1 | A6OH >7000 | NI | NI | NI |
| D2 | A6OH 7000-3000 | NI | NI | NI |
| D3 | A6OH <3000 | NI | NI | NI |
| E | I2OH, NAc | NI | NI | NI |
| E1 | I2OH, NAc >7000 | NI | NI | NI |
| E2 | I2OH, NAc 7000-3000 | NI | NI | NI |
| E3 | I2OH, NAc <3000 | NI | NI | NI |
| F | A6OH, NAc | NI | NI | NI |
| F1 | A6OH, NAc >7000 | NI | NI | NI |
| F2 | A6OH, NAc 7000-3000 | NI | NI | NI |
| F3 | A6OH, NAc <3000 | NI | NI | NI |
| G | I2OH, A6OH | NI | NI | NI |
| G1 | I2OH, A6OH >7000 | NI | NI | NI |
| G2 | I2OH, A6OH 7000-3000 | NI | NI | NI |
| G3 | I2OH, A6OH <3000 | NI | NI | NI |
| H | I2OH, A6OH, NAc | NI | NI | NI |
| H1 | I2OH, A6OH, NAc >7000 | NI | NI | NI |
| H2 | I2OH, A6OH, NAc 7000-3000 | NI | NI | NI |
| H3 | I2OH, A6OH, NAc <3000 | NI | NI | NI |

| | | | | |
|---|---|---|---|---|
| NI = no inhibition | | | | |

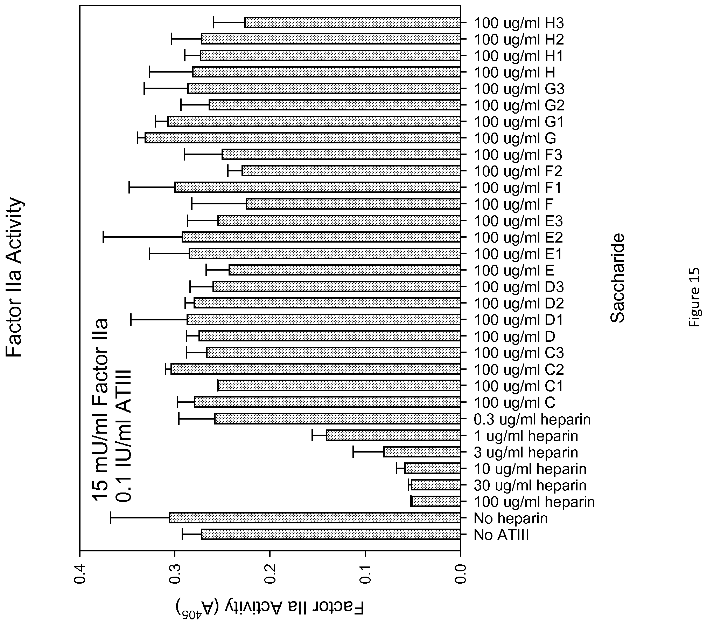

### Example 10 - Assessments of anti-coagulant activity of the heparin derivatives (Figure 15, Table 4)

Factor Xa and Factor II activity was measured using a colorimetric substrate assay as previously described (Guimond et al, 2006). Briefly, in a 96 well ELISA plate antithrombin III (American Diagnostica, 30 mIU/ml final concentration) was incubated with heparin or polysaccharide fractions in 0.9% NaCl at 37C for 2 minutes. Factor IIa (Sigma, 15 mU/ml final concentration) or Factor Xa (Thermo Scientific, 15 mU/ml final concentration) was added and the samples incubated a further 1 minute at 37C. Factor Xa Chromogenic substrate (Sigma, 240 uM final concentration) was added to the samples and incubated 10 minutes at 37C. Reaction was stopped with glacial acetic acid (Sigma, 25% final concentration). Color change of the substrate was measured at 405 nm. Activated Partial Thromboplastin Time (APTT) and Prothrombin Time (PT) assays were performed using a Axis Shield Thrombotrack 1 instrument using normal human plasma, Pathrombin SL reagent and Thromborel S (all from Axis Shield) reagents according to manufacturer's instructions. For all assays, heparins and polysaccharides were tested up to 100 ug/ml. Heparin was an anticoagulant in all assays with IC50 values of 0.9, 1.0, 1.2 and 41.4 ug/ml for the Factor Xa, Factor II, APTT and PT assays respectively (Table 2). Polysaccharides D and D1 were approximately 3 fold less active than heparin in the APTT assay but showed no anticoagulation in the other assays. No other polysaccharide fraction had anticoagulant activity (<1% of heparin activity).

### Results and Conclusion:

Most of the heparin derivatives, fractionated or non-fractionated (with the exception of D and D1), show anti-factor IIa activity (Figure 15 Table 4).

**Table 4 Anticoagulant activity of heparin and polysaccharide fractions**

| Compound | Structure | IC50 Factor Xa | IC50 Factor IIa | IC50 APTT | IC50 PT |
|---|---|---|---|---|---|
| Heparin | | 0.9295 | 1.0326 | 1.2461 | 41.4005 |
| C | I2OH | NI | NI | NI | NI |
| C1 | I2OH >7000 | NI | NI | NI | NI |
| C2 | I2OH 7000-3000 | NI | NI | NI | NI |
| C3 | I2OH <3000 | NI | NI | NI | NI |
| D | A6OH | NI | NI | 3.2996 | NI |
| D1 | A6OH >7000 | NI | NI | 3.1175 | NI |
| D2 | A6OH 7000-3000 | NI | NI | NI | NI |
| D3 | A6OH <3000 | NI | NI | NI | NI |
| E | I2OH, NAc | NI | NI | NI | NI |
| E1 | I2OH, NAc >7000 | NI | NI | NI | NI |
| E2 | I2OH, NAc 7000-3000 | NI | NI | NI | NI |
| E3 | I2OH, NAc <3000 | NI | NI | NI | NI |
| F | A6OH, NAc | NI | NI | NI | NI |
| F1 | A6OH, NAc >7000 | NI | NI | NI | NI |
| F2 | A6OH, NAc 7000-3000 | NI | NI | NI | NI |
| F3 | A6OH, NAc <3000 | NI | NI | NI | NI |
| G | I2OH, A6OH | NI | NI | NI | NI |
| G1 | I2OH, A6OH >7000 | NI | NI | NI | NI |
| G2 | I2OH, A6OH 7000-3000 | NI | NI | NI | NI |
| G3 | I2OH, A6OH <3000 | NI | NI | NI | NI |
| H | I2OH, A6OH, NAc | NI | NI | NI | NI |
| H1 | I2OH, A6OH, NAc >7000 | NI | NI | NI | NI |
| H2 | I2OH, A6OH, NAc 7000-3000 | NI | NI | NI | NI |
| H3 | I2OH, A6OH, NAc <3000 | NI | NI | NI | NI |

| | | | | | |
|---|---|---|---|---|---|
| NI = no inhibition | | | | | |

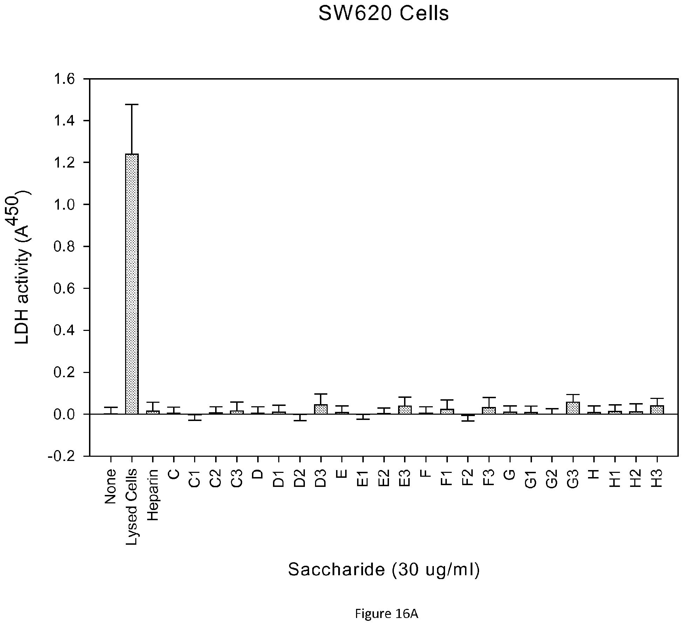

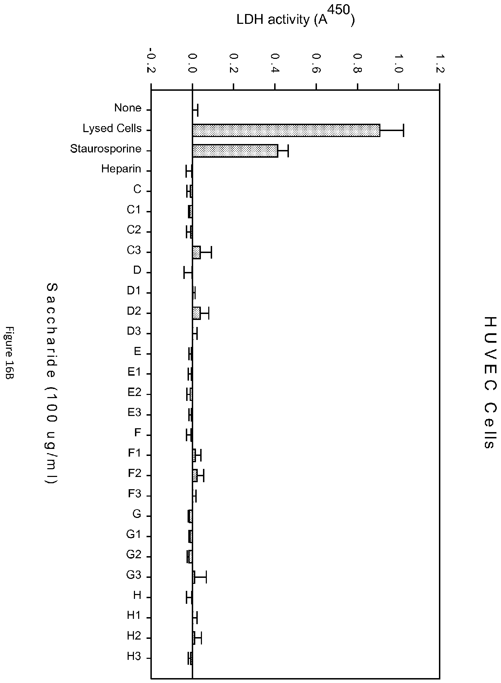

### Example 11 - Assessments of cytotoxicity of the heparin derivatives to cancer and endothelial cells (Figures 16A and 16B)

HUVEC, ACA19+ or SW620 cells were plated at 5000 cells/well in 100 µl of EBS+ medium (Lonza; for HUVEC cells) or DME medium (Life Technologies) supplemented with 10% FBS (Life Technologies; for ACA19+ and SW620 cells). Cells were incubated with 100 µg/ml of saccharides for 24 or 48 hours. Cell death was measured using an LDH-Cytotoxicity Assay Kit II (Abcam) per the manufacturer's instructions. None of the saccharides caused appreciable cell death compared to cells that had been treated with 1 µM staurosporine (Sigma) or Cell Lysis buffer supplied with the kit.

### Results and Conclusion:

None of the heparin derivatives, fractionated or non-fractionated, including E, E3, F and F3, show cytotoxicity to cancer or endothelial cells (Figures 16A and 16B).

### References

Guimond, S.E., Turnbull, J.E. and Yates, E.A. (2006) Engineered Bio-Active Polysaccharides from Heparin. Macromolecular Biosciences, 6: 681-686.
Liu, Z and Perlin, A.S. (1994) Evidence of a selective free radical degradation of heparin, mediated by cupric ion. Carbohydrate Research, 255: 183-191.
Nelson, R.M., Cecconi, O., Roberts, W.G., Aruffo, A., Linhardt, R.J. and Bevilacqua, M.P. (1993) Heparin oligosaccharides bind L- and P-selectin and inhibit acute inflammation. Blood, 82: 3253-3258

## Claims

1. A heparin derivative for use in the prevention and/or treatment of cancer and/or cancer metastasis, wherein said derivative comprises one or more disaccharide units comprising a uronate moiety linked to a glucosamine moiety, and wherein:
(i) the 2-0 atom of the uronate moiety and/or the 6-0 atom of the glucosamine moiety are substantially desulphated; and
(ii) the heparin derivative exhibits less than 1 % of the Anti-Factor Xa activity of unmodified porcine intestinal mucosal heparin;
or a pharmaceutically acceptable salt or solvate thereof

2. A heparin derivative for use according to claim 1, wherein the heparin derivatives exhibits less than around 0.5 % of the Anti-Factor Xa activity of unmodified porcine intestinal mucosal heparin.

3. A heparin derivative for use according to any one of the preceding claims, wherein 30 to 100% of the 2-O atoms on the uronate moieties and/or the 6-O atoms of the glucosamine moieties of the heparin molecule are substituted with hydrogen atoms.

4. A heparin derivative for use according to claim 3, wherein 75 to 100% of the 2-O atoms on the uronate moieties and/or the 6-O atoms of the glucosamine moieties of the heparin molecule are substituted with hydrogen atoms.

5. A heparin derivative for use according to any one of the preceding claims, wherein the 2-*N* atom of the glucosamine moiety is sulphated.

6. A heparin derivative for use according to any one of claims 1 to 4, wherein the 2-*N* atom of the glucosamine moiety is substantially desulphated.

7. A heparin derivative for use according to any one of claims 1 to 4, wherein substantially all of the 2-*N* atoms of the glucosamine moieties present are substituted with a substituent selected from hydrogen, substituted or unsubstituted (1-8C)alkyl, substituted or unsubstituted aryl, substituted or unsubstituted (2-8C)acyl, substituted or unsubstituted amido or phosphate.

8. A heparin derivative for use according to claim 1 or claim 2, wherein the heparin derivatives are:
(i) substantially 2-O desulphated and substantially 2-N desulphated (e.g. 2-N substituted) as defined herein;
(ii) substantially 6-O desulphated and substantially 2-N desulphated (e.g. 2-N substituted) as defined herein;
(iii) substantially 2-O desulphated and substantially 6-O desulphated as defined herein; or
(iv) substantially 2-O desulphated, substantially 6-O desulphated, and substantially 2-N desulphated (e.g. 2-N substituted)as defined herein.

9. A heparin derivative for use according to claim 1, wherein the heparin derivative has the general structural formula I shown below: 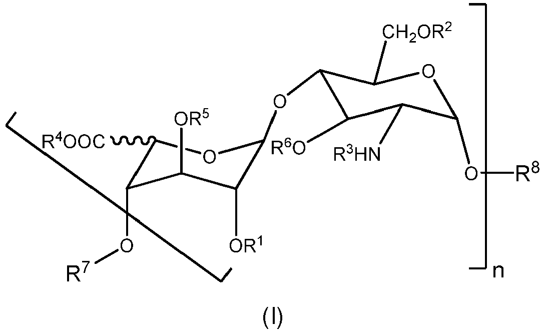 wherein:
R¹ and R² are selected from hydrogen or sulphate, with the proviso that either:
(i) substantially all of the R¹ groups present in the molecule are hydrogen when substantially all (e.g. >70%) of the R² groups present are sulphate;
(ii) substantially all of the R² groups present in the molecule are hydrogen when substantially all (e.g. >70%) of the R¹ groups present are sulphate, or
(iii) substantially all of the R¹ and R² groups present in the molecule are hydrogen;
n is 1 to 30;
R³ is selected from sulphate, hydrogen, substituted or unsubstituted alkyl, substituted or unsubstituted aryl, substituted or unsubstituted acyl, substituted or unsubstituted amido and phosphate;
R⁴ is selected from the group consisting of hydrogen, substituted or unsubstituted (1-6C)alkyl, substituted or unsubstituted aryl;
R⁵ and R⁶ are each separately selected from the group consisting of hydrogen, sulphate, phosphate, substituted or unsubstituted (1-6C)alkyl, substituted or unsubstituted (1-6C)alkoxy, substituted or unsubstituted aryl, substituted or unsubstituted aryloxy, substituted or unsubstituted acyl, and substituted or unsubstituted amido; and
R⁷ and R⁸ are each separately selected from the group consisting of hydrogen, substituted or unsubstituted alkyl, substituted or unsubstituted aryl, substituted or unsubstituted acyl, a terminal monosaccharide group, a terminal disaccharide group and/or fragments or derivatives thereof;
or a pharmaceutically acceptable salt thereof.

10. A heparin derivative for use according to claim 9, wherein
(i) substantially all of the R¹ groups are hydrogen (i.e. the molecule is 2-O desulphated as defined hereinbefore) and substantially all of the R³ groups present are hydrogen or a substituent other than sulphate as defined above (i.e. the molecule is 2-N desulphated (e.g. 2-N substituted) as defined hereinbefore);
(ii) substantially all of the R² groups are hydrogen (i.e. the molecule is 6-O desulphated as defined hereinbefore) and substantially all of the R³ groups present are hydrogen or a substituent other than sulphate as defined above (i.e. the molecule is 2-N desulphated (e.g. 2-N substituted) as defined hereinbefore);
(iii) substantially all of the R¹ and R² groups are hydrogen (i.e. the molecule is substantially 2-O and 6-O desulphated as defined herein); or
(iv) substantially all of the R¹ and R² groups are hydrogen (i.e. the molecule is substantially 2-O and 6-O desulphated as defined herein) and substantially all of the R³ groups present are hydrogen or a substituent other than sulphate as defined above (i.e. the molecule is 2-N desulphated (e.g. 2-N substituted) as defined hereinbefore).

11. A heparin derivative for use according to any one of the preceding claims, wherein the average molecular weight of the heparin derivatives ranges from 300 Da to 30 kDa.

12. A heparin derivative for use accoring claim 11, wherein the average molecular weight of the heparin derivatives of the invention will range from 500 Da to 3.5 kDa.

13. A heparin derivative for use according to any one of the preceding claims, wherein the degree of polymerisation of the heparin derivative ranges from 2 monomer units up to 60 monomer units.

14. A heparin derivative for use according to claim 13, wherein the degree of polymerisation of the heparin derivative ranges from 2 monomer units up to 7 monomer units.

## Patentansprüche

1. Heparinderivat zur Verwendung bei der Prävention und/oder Behandlung von Krebs und/oder Krebsmetastasen, wobei dieses Derivat eine oder mehrere Disaccharideinheiten umfasst, umfassend eine Uronatkomponente verbunden mit einer Glucosaminkomponente, und wobei:
(i) das 2-*O*-Atom der Uronatkomponente und/oder das 6-*O*-Atom der Glucosaminkomponente im Wesentlichen desulfatiert sind; und
(ii) das Heparinderivat weniger als 1 % der Anti-Faktor-Xa-Aktivität des unmodifizierten Heparins der Darmschleimhaut von Schweinen zeigt;
oder ein pharmazeutisch unbedenkliches Salz oder Solvat davon

2. Heparinderivat zur Verwendung nach Anspruch 1, wobei das Heparinderivat weniger als etwa 0,5 % der Anti-Faktor-Xa-Aktivität des unmodifizierten Heparins der Darmschleimhaut von Schweinen zeigt.

3. Heparinderivat zur Verwendung nach einem der vorstehenden Ansprüche, wobei 30 bis 100 % der 2-O-Atome an den Uronatkomponenten und/oder der 6-O-Atome der Glucosaminkomponenten des Heparinmoleküls durch Wasserstoffatome substituiert sind.

4. Heparinderivat zur Verwendung nach Anspruch 3, wobei 75 bis 100 % der 2-O-Atome an den Uronatkomponenten und/oder der 6-O-Atome der Glucosaminkomponenten des Heparinmoleküls durch Wasserstoffatome substituiert sind.

5. Heparinderivat zur Verwendung nach einem der vorstehenden Ansprüche, wobei das 2-*N-*Atom der Glucosaminkomponente sulfatiert ist.

6. Heparinderivat zur Verwendung nach einem der Ansprüche 1 bis 4, wobei das 2-*N*-Atom der Glucosaminkomponente im Wesentlichen desulfatiert ist.

7. Heparinderivat zur Verwendung nach einem der Ansprüche 1 bis 4, wobei im Wesentlichen alle 2-*N*-Atome der Glucosaminkomponenten, die vorliegen, durch einen Substituenten substituiert sind, der gewählt ist aus Wasserstoff, substituiertem oder unsubstituiertem (1-8C)Alkyl, substituiertem oder unsubstituiertem Aryl, substituiertem oder unsubstituiertem (2-8C)Acyl, substituiertem oder unsubstituiertem Amido oder Phosphat.

8. Heparinderivat zur Verwendung nach Anspruch 1 oder Anspruch 2, wobei die Heparinderivate:
(i) im Wesentlichen 2-O-desulfatiert und im Wesentlichen 2-N-desulfatiert (z. B. 2-N-substituiert) sind, wie hier definiert;
(ii) im Wesentlichen 6-O-desulfatiert und im Wesentlichen 2-N-desulfatiert (z. B. 2-N-substituiert) sind, wie hier definiert;
(iii) im Wesentlichen 2-O-desulfatiert und im Wesentlichen 6-O-desulfatiert sind, wie hier definiert; oder
(iv) im Wesentlichen 2-O-desulfatiert, im Wesentlichen 6-O-desulfatiert und im Wesentlichen 2-N-desulfatiert (z. B. 2-N-substituiert) sind, wie hier definiert.

9. Heparinderivat zur Verwendung nach Anspruch 1, wobei das Heparinderivat die nachstehend dargestellte allgemeine Strukturformel I aufweist: 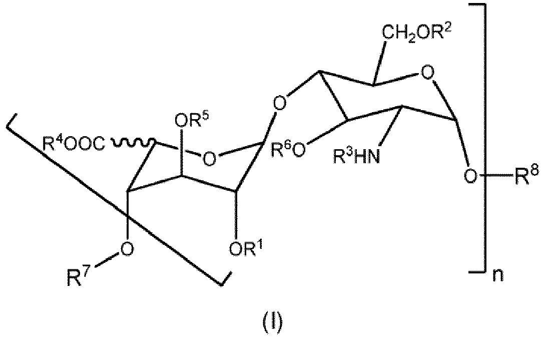 wobei:
R¹ und R² ausgewählt sind aus Wasserstoff oder Sulfat, mit der Maßgabe, dass entweder:
(i) im Wesentlichen alle R¹-Gruppen, die im Molekül vorliegen, Wasserstoff sind, wenn im Wesentlichen alle (z. B. >70 %) der R²-Gruppen, die vorliegen, Sulfat sind;
(ii) im Wesentlichen alle R²-Gruppen, die im Molekül vorliegen, Wasserstoff sind, wenn im Wesentlichen alle (z. B. >70 %) der R¹-Gruppen, die vorliegen, Sulfat sind, oder
(iii) im Wesentlichen alle R¹- und R²-Gruppen, die im Molekül vorliegen, Wasserstoff sind;
n 1 bis 30 ist;
R³ ausgewählt ist aus Sulfat, Wasserstoff, substituiertem oder unsubstituiertem Alkyl, substituiertem oder unsubstituiertem Aryl, substituiertem oder unsubstituiertem Acyl, substituiertem oder unsubstituiertem Amido und Phosphat;
R⁴ ausgewählt ist aus der Gruppe bestehend aus Wasserstoff, substituiertem oder unsubstituiertem (1-6C)Alkyl, substituiertem oder unsubstituiertem Aryl;
R⁵ und R⁶ jeweils getrennt ausgewählt sind aus der Gruppe bestehend aus Wasserstoff, Sulfat, Phosphat, substituiertem oder unsubstituiertem (1-6C)Alkyl, substituiertem oder unsubstituiertem (1-6C)Alkoxy, substituiertem oder unsubstituiertem Aryl, substituiertem oder unsubstituiertem Aryloxy, substituiertem oder unsubstituiertem Acyl und substituiertem oder unsubstituiertem Amido; und
R⁷ und R⁸ jeweils getrennt ausgewählt sind aus der Gruppe bestehend aus Wasserstoff, substituiertem oder unsubstituiertem Alkyl, substituiertem oder unsubstituiertem Aryl, substituiertem oder unsubstituiertem Acyl, einer terminalen Monosaccharidgruppe, einer terminalen Disaccharidgruppe und/oder Fragmenten oder Derivaten davon;
oder ein pharmazeutisch verträgliches Salz davon.

10. Heparinderivat zur Verwendung nach Anspruch 9, wobei
(i) im Wesentlichen alle R¹-Gruppen Wasserstoff sind (d. h. das Molekül ist 2-O-desulfatiert, wie vorstehend definiert) und im Wesentlichen alle R³-Gruppen, die vorliegen, Wasserstoff oder ein von Sulfat verschiedener Substituent sind, wie oben definiert (d. h. das Molekül ist 2-N-desulfatiert (z. B. 2-N-substituiert), wie vorstehend definiert);
(ii) im Wesentlichen alle R²-Crruppen Wasserstoff sind (d. h. das Molekül ist 6-O-desulfatiert, wie vorstehend definiert) und im Wesentlichen alle R³-Gruppen, die vorliegen, Wasserstoff oder ein von Sulfat verschiedener Substituent sind, wie oben definiert (d. h. das Molekül ist 2-N-desulfatiert (z. B. 2-N-substituiert), wie vorstehend definiert);
(iii) im Wesentlichen alle R¹- und R²-Gruppen Wasserstoff sind (d. h. das Molekül ist im Wesentlichen 2-O- und 6-O-desulfatiert, wie hier definiert); oder
(iv) im Wesentlichen alle R¹- und R²-Gruppen Wasserstoff sind (d. h. das Molekül ist im Wesentlichen 2-O- und 6-O-desulfatiert, wie hier definiert) und im Wesentlichen alle R³-Gruppen, die vorliegen, Wasserstoff oder ein von Sulfat verschiedener Substituent sind, wie oben definiert (d. h. das Molekül ist 2-N-desulfatiert (z. B. 2-N-substituiert), wie vorstehend definiert).

11. Heparinderivat zur Verwendung nach einem der vorstehenden Ansprüche, wobei die durchschnittliche Molekülmasse der Heparinderivate von 300 Da bis 30 kDa reicht.

12. Heparinderivat zur Verwendung nach Anspruch 11, wobei die durchschnittliche Molekülmasse der Heparinderivate der Erfindung von 500 Da bis 3,5 kDa reicht.

13. Heparinderivat zur Verwendung nach einem der vorstehenden Ansprüche, wobei der Polymerisationsgrad des Heparinderivats von 2 Monomereinheiten bis zu 60 Monomereinheiten reicht.

14. Heparinderivat zur Verwendung nach Anspruch 13, wobei der Polymerisationsgrad des Heparinderivats von 2 Monomereinheiten bis zu 7 Monomereinheiten reicht.

## Revendications

1. Dérivé d'héparine destiné à être utilisé dans la prévention et/ou le traitement du cancer et/ou d'une métastase cancéreuse, ledit dérivé comprenant un ou plusieurs motifs disaccharidiques comprenant un groupement uronate lié à un groupement glucosamine, et
(i) l'atome 2-*O* du groupement uronate et/ou l'atome 6-*O* du groupement glucosamine étant pratiquement désulfatés ; et
(ii) le dérivé d'héparine présentant moins de 1 % de l'activité anti-facteur Xa de l'héparine de muqueuse intestinale de porc non modifiée ;
ou sel ou solvate pharmaceutiquement acceptable de celui-ci.

2. Dérivé d'héparine destiné à être utilisé selon la revendication 1, ledit dérivé d'héparine présentant moins d'environ 0,5 % de l'activité anti-facteur Xa de l'héparine de muqueuse intestinale de porc non modifiée.

3. Dérivé d'héparine destiné à être utilisé selon l'une quelconque des revendications précédentes, 30 à 100 % des atomes 2-O sur les groupements uronate et/ou des atomes 6-O des groupements glucosamine de la molécule d'héparine étant substitués par des atomes d'hydrogène.

4. Dérivé d'héparine destiné à être utilisé selon la revendication 3, 75 à 100 % des atomes 2-O sur les groupements uronate et/ou des atomes 6-O des groupements glucosamine de la molécule d'héparine étant substitués par des atomes d'hydrogène.

5. Dérivé d'héparine destiné à être utilisé selon l'une quelconque des revendications précédentes, ledit atome 2-N du groupement glucosamine étant sulfaté.

6. Dérivé d'héparine destiné à être utilisé selon l'une quelconque des revendications 1 à 4, ledit atome 2-N du groupement glucosamine étant pratiquement désulfaté.

7. Dérivé d'héparine destiné à être utilisé selon l'une quelconque des revendications 1 à 4, pratiquement tous les atomes 2-N des groupements glucosamine présents étant substitués par un substituant choisi parmi un atome d'hydrogène, un groupe alkyle en C₁ à C₈ substitué ou non substitué, aryle substitué ou non substitué, acyle en C₂ à C₈ substitué ou non substitué, amido substitué ou non substitué ou phosphate.

8. Dérivé d'héparine destiné à être utilisé selon l'une quelconque des revendications 1 ou 2, lesdits dérivés d'héparine étant :
(i) pratiquement 2-O désulfatés et pratiquement 2-N désulfatés (par ex. 2-N substitués) tels que définis ici ;
(ii) pratiquement 6-O désulfatés et pratiquement 2-N désulfatés (par ex. 2-N substitués) tels que définis ici ;
(iii) pratiquement 2-O désulfatés et pratiquement 6-O désulfatés tels que définis ici ; ou
(iv) pratiquement 2-O désulfatés, pratiquement 6-O désulfatés et pratiquement 2-N désulfatés (par ex. 2-N substitués) tels que définis ici.

9. Dérivé d'héparine destiné à être utilisé selon la revendication 1, ledit dérivé d'héparine ayant la formule structurelle générale I présentée ci-dessous : 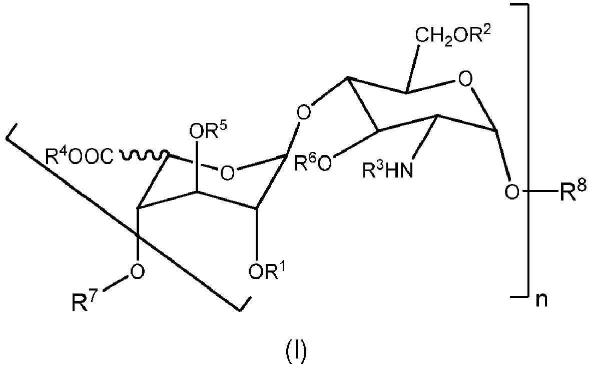 dans laquelle :
R¹ et R² sont choisis parmi l'atome d'hydrogène ou de sulfate, à condition que soit :
(i) pratiquement tous les groupes R¹ présents dans la molécule représentent un atome d'hydrogène lorsque pratiquement tous (par ex. > 70 %) des groupes R² présents représentent un atome de sulfate ; soit
(ii) pratiquement tous les groupes R² présents dans la molécule représentent un atome d'hydrogène lorsque pratiquement tous (par ex. > 70 %) des groupes R¹ présents représentent un atome de sulfate ; soit
(iii) pratiquement tous les groupes R¹ et R² présents dans la molécule représentent un atome d'hydrogène
n vaut de 1 à 30 ;
R³ est choisi parmi un atome de sulfate, d'hydrogène, un groupe alkyle substitué ou non substitué, aryle substitué ou non substitué, acyle substitué ou non substitué, amido substitué ou non substitué et phosphate ;
R⁴ est choisi dans le groupe constitué par un atome d'hydrogène, un groupe alkyle en C₁ à C₆ substitué ou non substitué, aryle substitué ou non substitué ;
R⁵ et R⁶ sont choisis chacun séparément dans le groupe constitué par un atome d'hydrogène, de sulfate, un groupe phosphate, un groupe alkyle en C₁ à C₆ substitué ou non substitué, alcoxy en C₁ à C₆ substitué ou non substitué, aryle substitué ou non substitué, aryloxy substitué ou non substitué, acyle substitué ou non substitué, et amido substitué ou non substitué ; et
R⁷ et R⁸ sont choisis chacun séparément dans le groupe constitué par un atome d'hydrogène, un groupe alkyle substitué ou non substitué, aryle substitué ou non substitué, acyle substitué ou non substitué, un groupe monosaccharide terminal, un groupe disaccharide terminal et/ou des fragments ou dérivés de ceux-ci ;
ou sel pharmaceutiquement acceptable de celui-ci.

10. Dérivé d'héparine destiné à être utilisé selon la revendication 9,
(i) pratiquement tous les groupes R¹ représentant un atome d'hydrogène (c'est-à-dire que la molécule est 2-O désulfatée telle que définie ci-dessus) et pratiquement tous les groupes R³ présents représentant un atome d'hydrogène ou un substituant différent d'un atome de sulfate tels que définis ci-dessus (c'est-à-dire que la molécule est 2-N désulfatée (par ex. 2-N substituée) telle que définie ci-dessus) ;
(ii) pratiquement tous les groupes R² représentant un atome d'hydrogène (c'est-à-dire que la molécule est 6-O désulfatée telle que définie ci-dessus) et pratiquement tous les groupes R³ présents représentant un atome d'hydrogène ou un substituant différent d'un atome de sulfate tels que définis ci-dessus (c'est-à-dire que la molécule est 2-N désulfatée (par ex. 2-N substituée) telle que définie ci-dessus) ;
(iii) pratiquement tous les groupes R¹ et R² représentant un atome d'hydrogène (c'est-à-dire que la molécule est pratiquement 2-O et 6-O désulfatée telle que définie ici) ; ou
(iv) pratiquement tous les groupes R¹ et R² représentant un atome d'hydrogène (c'est-à-dire que la molécule est 2-O et 6-O désulfatée telle que définie ici) et pratiquement tous les groupes R³ présents représentant un atome d'hydrogène ou un substituant différent d'un atome de sulfate tels que définis ci-dessus (c'est-à-dire que la molécule est 2-N désulfatée (par ex. 2-N substituée) telle que définie ci-dessus).

11. Dérivé d'héparine destiné à être utilisé selon l'une quelconque des revendications précédentes, le poids moléculaire moyen des dérivés d'héparine allant de 300 Da à 30 kDa.

12. Dérivé d'héparine destiné à être utilisé selon la revendication 11, ledit poids moléculaire moyen des dérivés d'héparine de l'invention allant de 500 Da à 3,5 kDa.

13. Dérivé d'héparine destiné à être utilisé selon l'une quelconque des revendications précédentes, le degré de polymérisation du dérivé d'héparine allant de 2 motifs monomères jusqu'à 60 motifs monomères.

14. Dérivé d'héparine destiné à être utilisé selon la revendication 13, ledit degré de polymérisation du dérivé d'héparine allant de 2 motifs monomères jusqu'à 7 motifs monomères.
